# EUROPÄISCHE PATENTSCHRIFT

(11) **EP 1 883 688 B1**
(45) Veröffentlichungstag und Bekanntmachung des Hinweises auf die Patenterteilung: **11.04.2018**
(21) Anmeldenummer: 06724488.9
(22) Anmeldetag: 21.04.2006
(51) Int. Cl.: C09K 11/06, H01L 51/50

(54) **VERBINDUNGEN FÜR ORGANISCHE ELEKTRONISCHE VORRICHTUNGEN**
COMPOUNDS FOR ORGANIC ELECTRONIC DEVICES
COMPOSES POUR DISPOSITIFS ELECTRONIQUES ORGANIQUES

(30) Priorität: 20.05.2005 DE 102005023437
(43) Veröffentlichungstag der Anmeldung: 06.02.2008
(73) Patentinhaber: Merck Patent GmbH, 64293 Darmstadt (DE)
(72) Erfinder: VESTWEBER, Horst, 34630 Gilersberg-Winterscheid (DE); HEIL, Holger, 64295 Darmstadt (DE); STOESSEL, Philipp, 60487 Frankfurt/Main (DE); BUESING, Arne, 65959 Frankfurt/Main (DE); PARHAM, Amir, Hossain, 65929 Frankfurt/Main (DE); FORTTE, Rocco, 65933 Frankfurt/Main (DE)
(86) Internationale Anmeldenummer: PCT/EP2006/003670
(87) Internationale Veröffentlichungsnummer: WO 2006/122630

(56) Entgegenhaltungen:
- PATENT ABSTRACTS OF JAPAN Bd. 2000, Nr. 04, 31. August 2000 (2000-08-31) -& JP 2000 026324 A (MITSUI CHEMICALS INC), 25. Januar 2000 (2000-01-25)
- XIAO-YU ET AL: THE JOURNAL OF ORGANIC CHEMISTRY, Bd. 69, Nr. 18, 3. September 2004 (2004-09-03), Seiten 6050-6058, XP002391238

## Beschreibung

Die vorliegende Erfindung beschreibt neue Verbindungen und deren Einsatz in organischen elektronischen Vorrichtungen.

Der allgemeine Aufbau organischer Elektrolumineszenzvorrichtungen ist beispielsweise in US 4539507, US 5151629, EP 0676461 und WO 98/27136 beschrieben. Allerdings zeigen diese Vorrichtungen immer noch erhebliche Probleme, die einer dringenden Verbesserung bedürfen:
1. Die Effizienz ist gerade bei fluoreszierenden OLEDs immer noch zu niedrig und muss verbessert werden.
2. Die operative Lebensdauer ist insbesondere bei blauer Emission immer noch gering, so dass bisher nur einfache Anwendungen kommerziell realisiert werden konnten.
3. Die Betriebsspannung ist gerade bei fluoreszierenden OLEDs recht hoch und sollte daher weiter verringert werden, um die Leistungseffizienz zu verbessern. Das ist insbesondere für mobile Anwendungen von großer Bedeutung.
4. Viele blau emittierende Emitter, die sowohl aromatische Amine, wie auch Vinylgruppen enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich.
5. Bei Lochtransportmaterialien gemäß dem Stand der Technik ist die Spannung abhängig von der Schichtdicke der Lochtransportschicht. In der Praxis wäre häufig eine dickere Schichtdicke der Lochtransportschicht wünschenswert. Dies lässt sich jedoch wegen des damit verbundenen Spannungsanstiegt mit Materialien gemäß dem Stand der Technik nicht realisieren.

Als nächstliegender Stand der Technik kann die Verwendung bestimmter Arylvinylamine von Idemitsu genannt werden (z. B. WO 04/013073, WO 04/016575, WO 04/018587). Damit werden sehr gute Lebensdauern bei tiefblauer Emission zitiert. Allerdings sind diese Ergebnisse stark abhängig vom verwendeten Hostmaterial, so dass die zitierten Lebensdauern nicht als Absolutwerte verglichen werden können, sondern immer nur bei Einsatz in einem optimierten System. Weiterhin sind diese Verbindungen thermisch instabil und lassen sich nicht unzersetzt verdampfen, was daher einen hohen technischen Aufwand für die OLED Herstellung erfordert und somit einen deutlichen technischen Nachteil darstellt. Einen weiteren Nachteil stellt die Emissionsfarbe dieser Verbindungen dar. Während Idemitsu tiefblaue Emission (CIE-y-Koordinaten im Bereich von 0.15-0.18) zitiert, konnten diese Farbkoordinaten nicht in einfachen Vorrichtungen gemäß dem Stand der Technik reproduziert werden. Im Gegenteil erhält man hier grünblaue Emission. Es ist nicht offensichtlich, wie mit diesen Verbindungen tatsächlich blaue Emission erzeugt werden kann.

Weitere Beispiele der blaulichtemittierenden organischen Verbindungen sind in US 2004/0131880 A1 zu finden. Es besteht also weiterhin ein Bedarf an blau emittierenden Verbindungen, die in organischen Elektrolumineszenzvorrichtungen zu guten Effizienzen und gleichzeitig zu hohen Lebensdauern führen und die technisch unproblematisch zu verarbeiten sind. Es wurde nun überraschend gefunden, dass organische Elektrolumineszenzvorrichtungen, die bestimmte - im Folgenden aufgeführte - Verbindungen als blau emittierende Dotanden in einem Hostmaterial enthalten, deutliche Verbesserungen gegenüber dem Stand der Technik aufweisen. Mit diesen Materialien ist es möglich, längere Lebensdauern bei höherer Effizienz zu erhalten. Außerdem lassen sich diese Verbindungen im Gegensatz zu Materialien gemäß dem Stand der Technik ohne merkliche Zersetzung auch in größeren Mengen sublimieren und sind daher deutlich leichter zu handhaben als Materialien gemäß dem Stand der Technik. Diese Verbindungen und deren Verwendung in OLEDs sind daher Gegenstand der vorliegenden Erfindung.

Gegenstand der Erfindung sind Verbindungen gemäß Formel (1), 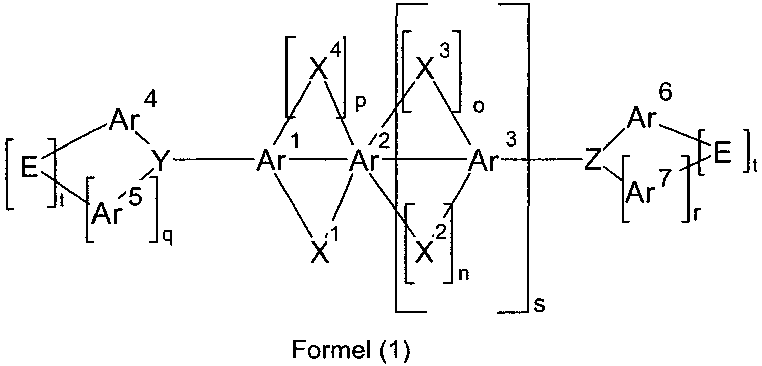 wobei für die verwendeten Symbole und Indizes gilt:
- Y, Z: ist gleich oder verschieden N, P, P=O, PF₂, P=S, As, As=O, As=S, Sb, Sb=O, Sb=S, Bi, Bi=O, Bi=S, C=O, O, S, Se, Te, S=O, SO₂, Se=O, SeO₂ Te=O oder TeO₂;
- Ar¹, Ar²,: ist Benzol, das mit einem oder mehreren Resten R¹ substituiert sein kann;
- Ar⁴, Ar⁵, Ar⁶, Ar⁷: ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
- E: ist bei jedem Auftreten gleich oder verschieden eine Einfachbindung, N(R¹), O, S, C(R¹)₂, Si(R¹)₂ oder B(R¹);
- R¹: ist bei jedem Auftreten gleich oder verschieden H, F, Cl, Br, I, CN, NO₂, B(OR²)₂, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -C≡C-, Si(R²)₂, Ge(R²)₂, Sn(R²)₂, C=O, C=S, C=Se, C=NR², -O-, -S-, -COO- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das durch einen oder mehrere nicht-aromatische Reste R¹ substituiert sein kann, oder eine Aryloxy- oder Heteroaryloxygruppe mit 5 bis 40 aromatischen Ringatomen, die durch einen oder mehrere nicht-aromatische Reste R¹ substituiert sein kann, oder eine Kombination dieser Systeme; dabei können zwei oder mehrere Substituenten R¹ auch miteinander ein mono- oder polycyclisches Ringsystem bilden;
- R²: ist bei jedem Auftreten gleich oder verschieden H oder ein aliphatischer oder aromatischer Kohlenwasserstoffrest mit 1 bis 20 C-Atomen;
- X¹, X⁴: ist bei jedem Auftreten gleich oder verschieden eine Brücke, die mit Ar¹ und Ar² ein cyclisches System aufspannt, ausgewählt aus B(R¹), C(R¹)₂, Si(R¹)₂, C=O, C=NR¹, C=C(R¹)₂, O, S, S=O, SO₂, N(R¹), P(R¹), P(=O)R¹, P(=S)R¹ oder einer Kombination aus zwei, drei oder vier dieser Gruppen;
- X², X³: ist bei jedem Auftreten gleich oder verschieden eine Brücke, die mit Ar² und Ar³ ein cyclisches Ringsystem aufspannt, ausgewählt aus B(R¹), C(R¹)₂, Si(R¹)₂, C=O, C=NR¹, C=C(R¹)₂, O, S, S=O, SO₂, N(R¹), P(R¹), P(=O)R¹, P(=S)R¹ oder einer Kombination aus zwei, drei oder vier dieser Gruppen;
- n, o, p: p ist null und einer der beiden Indizes n oder o ist eins, während der andere der beiden Indizes n oder o null ist, dabei bedeutet n = 0 bzw. o = 0 bzw. p = 0, dass sich an Stelle der Brücke zwei H bzw. Reste R¹ befinden;
- q, r: ist bei jedem Auftreten 1, wenn das entsprechende Zentralatom der Gruppe Y bzw. Z ein Element aus der 5. Hauptgruppe ist, und ist bei jedem Auftreten gleich 0, wenn das entsprechende Zentralatom der Gruppe Y bzw. Z ein Element aus der 4. oder der 6. Hauptgruppe ist;
- s: ist 1, 2 oder 3;
- t: ist bei jedem Auftreten gleich oder verschieden 0 oder 1, wobei t = 0 bedeutet, dass statt der Gruppe E Reste R¹ gebunden sind; dabei gilt weiterhin, dass t = 0 ist, wenn q = 0 ist.

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden, wobei eine Arylgruppe 6 bis 24 C-Atome und eine Heteroarylgruppe 2 bis 24 C-Atome und insgesamt mindestens 5 aromatische Ringatome umfasst. Die Heteroatome sind bevorzugt ausgewählt aus N, O und/oder S. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. Diese Aryl- oder Heteroarylgruppe kann substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe der C-Atome und Heteroatome mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen und heteroaromatischen Ringsystemen im Sinne dieser Erfindung werden außer den oben genannten Aryl- und Heteroarylgruppen insbesondere Biphenylen, Terphenylen, Fluoren, Spirobifluoren, Dihydrophenanthren, Tetrahydropyren und cis- oder trans-Indenofluoren verstanden. Bevorzugt sind Verbindungen gemäß Formel (1), in denen die Symbole Y und Z gleich oder verschieden für Stickstoff, C=O, Phosphor oder P=O stehen, besonders bevorzugt für Stickstoff, C=O oder P=O. Ganz besonders bevorzugt stehen Y und Z für Stickstoff.

Die direkte Verknüpfung zwischen Y, Ar¹, Ar², Ar³ und Z erfolgt besonders bevorzugt über die para-Positonen des Benzols (bzw. die entsprechenden Positionen der anderen Aromaten).

Besonders bevorzugt sind also Verbindungen der Formel (1a), 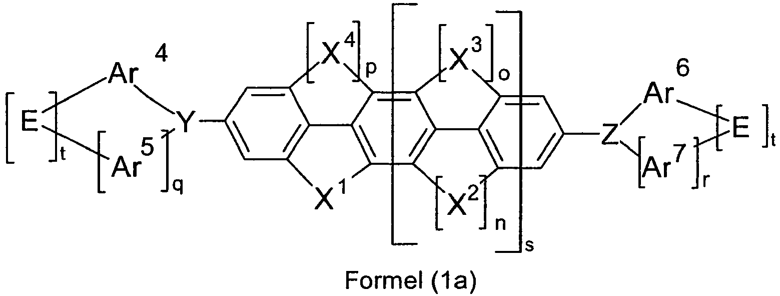 wobei die Symbole und Indizes dieselben Bedeutungen haben, wie oben beschrieben.

Bevorzugt sind weiterhin Verbindungen gemäß Formel (1) bzw. (1a), in denen die Symbole Ar⁴, Ar⁵, Ar⁶ und Ar⁷ gleich oder verschieden bei jedem Auftreten für ein aromatisches oder heteroaromatisches Ringsystem mit 5 bis 16 aromatischen Ringatomen, für ein Triarylamin oder für Spirobifluoren stehen, das jeweils mit einem oder mehreren Resten R¹ substituiert sein kann, besonders bevorzugt für ein aromatisches oder heteroaromatisches Ringsystem, ausgewählt aus Benzol, Naphthalin, Anthracen, Phenanthren, Pyridin, Pyren, Thiophen, Triphenylamin, Diphenyl-1-naphthylamin, Diphenyl-2-naphthylamin, Phenyl-di(1-naphthyl)amin und Phenyl-di(2-naphthyl)amin, welches jeweils mit R¹ substituiert sein kann. Ganz besonders bevorzugt stehen die Symbole Ar⁴, Ar⁵, Ar⁶ und Ar⁷ gleich oder verschieden bei jedem Auftreten für Phenyl, 1-Naphthyl oder 2-Naphthyl, das jeweils mit einem oder zwei Resten R¹ substituiert sein kann.

Bevorzugt sind weiterhin Verbindungen gemäß Formel (1) bzw. (1a), in denen der Index t = 0 ist oder in denen der Index t = 1 ist und das entsprechende Symbol E für eine Einfachbindung, O, S oder N(R¹) steht. Ganz besonders bevorzugt sind Verbindungen gemäß Formel (1) bzw. (1a), in denen der Index t = 0 ist oder in denen der Index t = 1 ist und das entsprechende Symbol E für eine Einfachbindung steht.

Bevorzugt sind weiterhin Verbindungen gemäß Formel (1), in denen das Symbol R¹ gleich oder verschieden bei jedem Auftreten für H, F, CN, eine geradkettige Alkylgruppe mit 1 bis 5 C-Atomen oder eine verzweigte Alkylgruppe mit 3 bis 5 C-Atomen, wobei jeweils eine oder mehrere nicht benachbarte CH₂-Gruppen durch -C≡C-, -O- oder -S- ersetzt sein können und wobei ein oder mehrere H-Atome durch F ersetzt sein können, oder eine monovalente Aryl- oder Heteroarylgruppe mit 5 bis 16 aromatischen Ringatomen, die mit einem oder mehreren nicht-aromatischen Resten R¹ substituiert sein kann, steht, wobei zwei oder mehrere Reste R¹ miteinander ein Ringsystem bilden können; besonders bevorzugt steht R¹ für H, F, CN, Methyl, tert-Butyl oder eine monovalente Aryl- oder Heteroarylgruppe mit 4 bis 6 C-Atomen, die mit einem oder mehreren nicht-aromatischen Resten R¹ substituiert sein kann, wobei zwei aromatische Reste R¹ miteinander ein Ringsystem bilden können. Ganz besonders bevorzugt sind R¹ = H, wenn es direkt an eine der Gruppen Ar¹ bis Ar⁷ gebunden ist.

Weiterhin bevorzugt ist R¹, wenn es an eine Gruppe X¹, X², X³ und/oder X⁴ gebunden ist bevorzugt eine geradkettige Alkylgruppe mit 1 bis 10 C-Atomen oder eine verzweigte oder cyclische Alkylgruppe mit 3 bis 10 C-Atomen, wobei jeweils eine oder mehrere nicht benachbarte CH₂-Gruppen durch -C≡C-, -O- oder -S- ersetzt sein können und wobei ein oder mehrere H-Atome durch F ersetzt sein können, oder eine monovalente Aryl- oder Heteroarylgruppe mit 5 bis 16 aromatischen Ringatomen, die mit einem oder mehreren nicht-aromatischen Resten R¹ substituiert sein kann; dabei können auch zwei Reste R¹ miteinander ein Ringsystem bilden.

Die Indizes p, n und o sind wie in Formel (1) definiert, bevorzugt sind p und n = 0 und o = 1.

Besonders bevorzugt sind daher die im Folgenden abgebildeten Strukturen gemäß Formel (1b) und (1c), insbesondere gemäß Formel (1c), 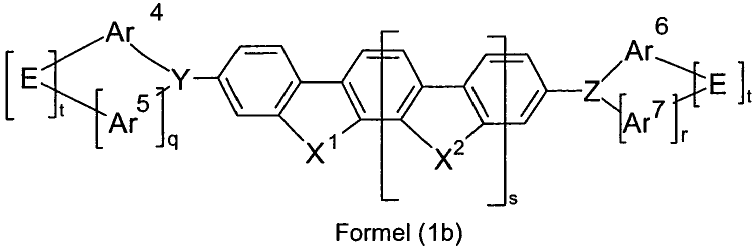 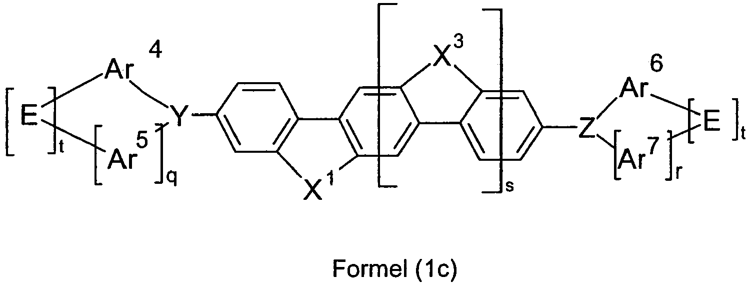 wobei die Symbole und Indizes dieselbe Bedeutung haben, wie oben beschrieben.

Bevorzugt sind weiterhin Verbindungen gemäß Formel (1) bzw. (1a) bis (1c), in denen die Symbole X¹, X², X³ und X⁴ bei jedem Auftreten gleich oder verschieden eine Brücke sind, die mit Ar¹ und Ar² bzw. mit Ar² und Ar³ ein cyclisches System aufspannt, ausgewählt aus C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O, C(R¹)₂-O-C(R¹)₂. Ganz besonders bevorzugt sind Verbindungen gemäß Formel (1), in denen die Symbole X¹, X², X³ und X⁴ bei jedem Auftreten gleich oder verschieden ausgewählt sind aus C(R¹)₂, N(R¹), P(R¹) und P(=O)(R¹), ganz besonders bevorzugt C(R¹)₂ oder N(R¹), insbesondere C(R¹)₂.

Ganz besonders bevorzugt sind daher Verbindungen der Formel (1d), 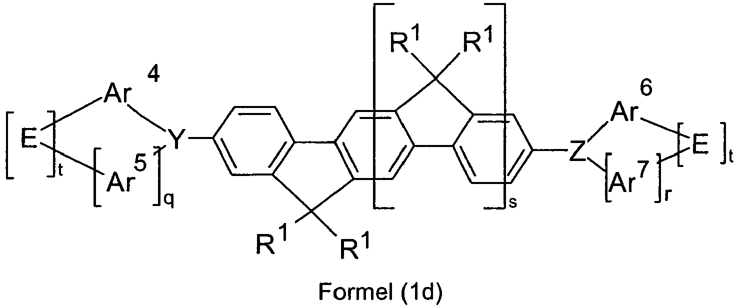 wobei die Symbole und Indizes dieselbe Bedeutung haben, wie oben beschrieben.

Bevorzugt sind in Strukturen der Formel (1d) die Symbole R¹ ausgewählt aus geradkettigen Alkylgruppen mit 1 bis 10 C-Atomen oder verzweigten oder cyclischen Alkylgruppen mit 3 bis 10 C-Atomen, wobei jeweils eine oder mehrere nicht benachbarte CH₂-Gruppen durch -C≡C-, -O- oder -S- ersetzt sein können und wobei ein oder mehrere H-Atome durch F ersetzt sein können, oder monovalenten Aryl- oder Heteroarylgruppen mit 5 bis 16 aromatischen Ringatomen, die mit einem oder mehreren nicht-aromatischen Resten R¹ substituiert sein können; dabei können auch zwei Reste R¹ miteinander ein Ringsystem bilden. Besonders bevorzugt sind in die Reste R¹ ausgesucht aus geradkettigen Alkylgruppen mit 1 bis 4 C-Atomen oder verzweigten Alkylgruppen mit 3 oder 4 C-Atomen, insbesondere Methylgruppen, oder Phenylgruppen; dabei können zwei oder mehrere Reste R¹ miteinander ein Ringsystem bilden.

Wenn mehrere Reste R¹ miteinander ein Ringsystem bilden, wird hierdurch eine Spiro-Struktur gebildet. Dies kann insbesondere dann bevorzugt sein, wenn die Reste R¹ für Phenylgruppen stehen. Dadurch ergeben sich dann Strukturen der allgemeinen Formel (1e), 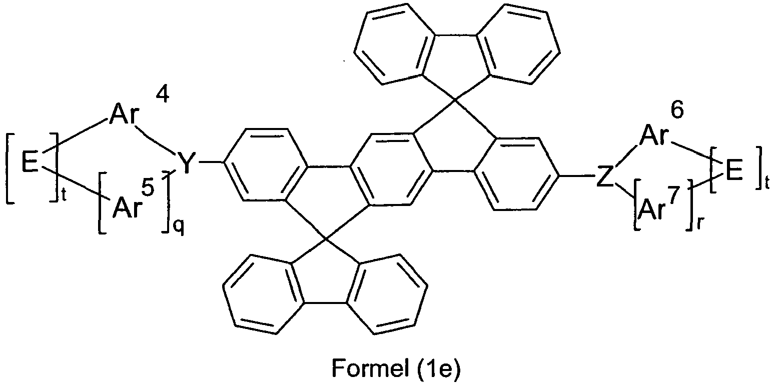 wobei die Symbole und Indizes dieselben Bedeutungen haben, wie oben beschrieben, und wobei die Spiro-Systeme jeweils durch einen oder mehrere nicht-aromatische Reste R¹ substituiert sein können.

Bevorzugt sind weiterhin Verbindungen gemäß Formel (1) bzw. Formel (1a) bis (1d), in denen das Symbol s=1 oder s=2 ist. Ganz besonders bevorzugt sind Verbindungen mit s=1.

Bevorzugt sind weiterhin Verbindungen gemäß Formel (1) bzw. (1a) bis (1e), in denen Y=Z ist. Ganz besonders bevorzugt sind Verbindungen, in denen zusätzlich Ar⁴ = Ar⁶ und, falls vorhanden, Ar⁵= Ar⁷ ist und, falls vorhanden, beide Gruppen E gleich gewählt sind.

Beispiele für bevorzugte Verbindungen gemäß Formel (1) sind die im Folgenden abgebildeten Strukturen (1) bis (104).

Beispiele 35 bis 41, 44, 45, 69, 75, 77, 78 und 81 bis 83 sind nicht gemäß Anspruch 1.

| | |
|---|---|
| 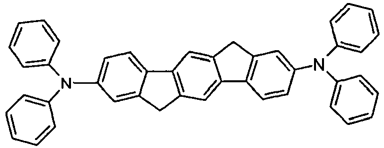 | 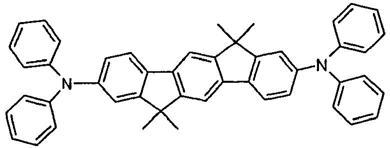 |
| (1) | (2) |
| 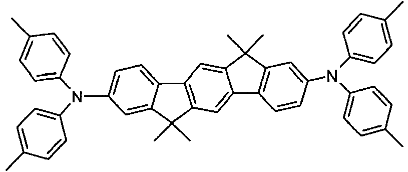 | 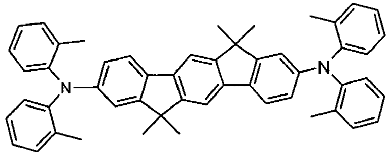 |
| (3) | (4) |
| 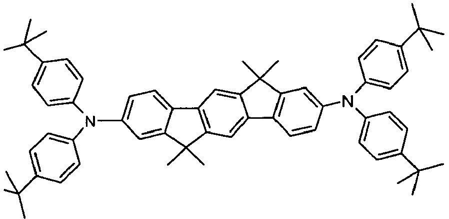 | 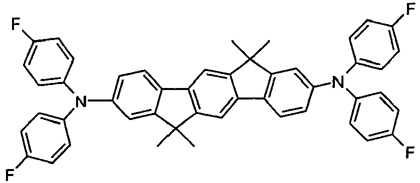 |
| (5) | (6) |
| 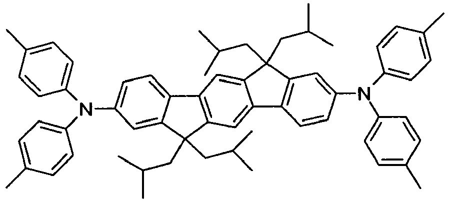 | 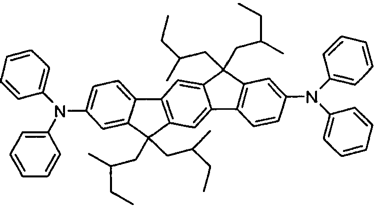 |
| (7) | (8) |
| 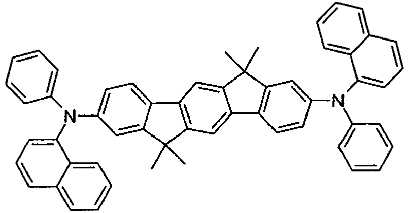 N | 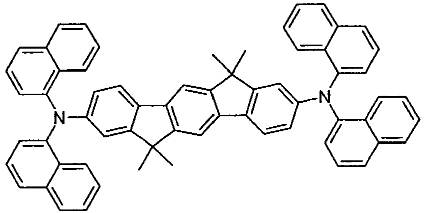 |
| (9) | (10) |
| 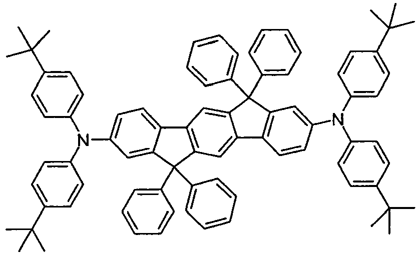 (9) | 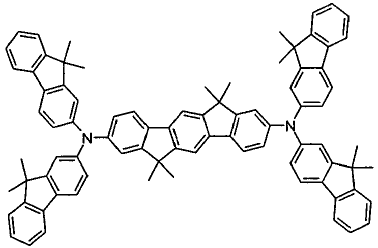 |
| (11) | (12) |
| 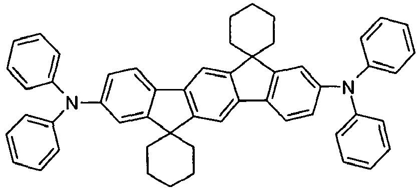 | 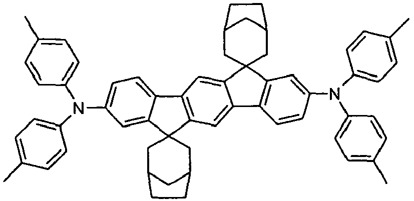 |
| (13) | (14) |
| 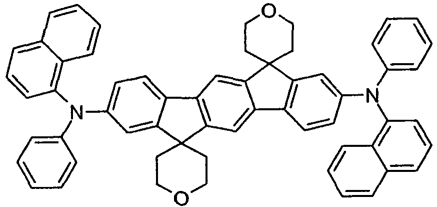 | 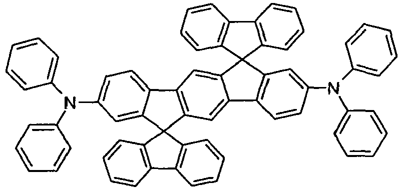 |
| (15) | (16) |
| 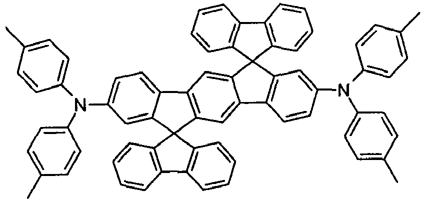 | 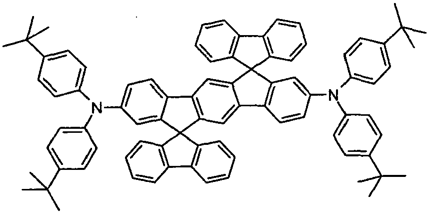 |
| (17) | (18) |
| 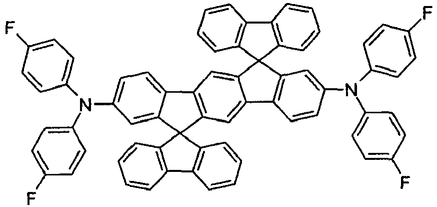 | 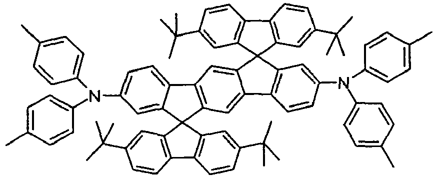 |
| (19) | (20) |
| 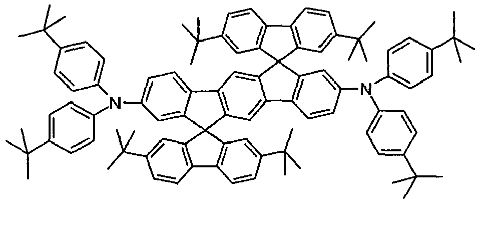 | 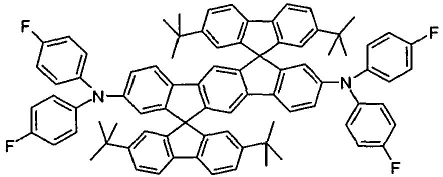 |
| (21) | (22) |
| 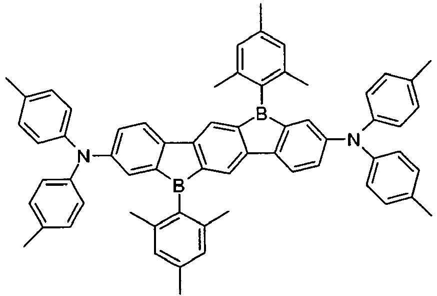 | 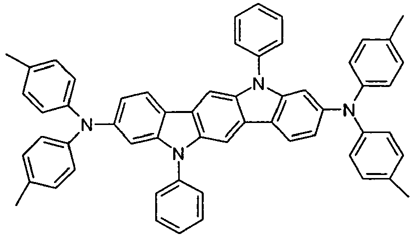 |
| (23) | (24) |
| 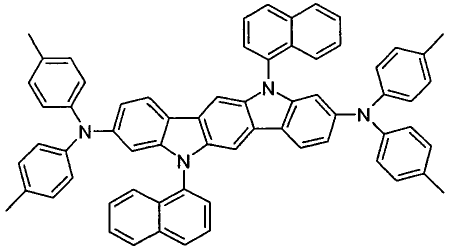 | 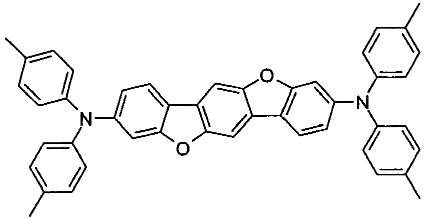 |
| (25) | (26) |
| 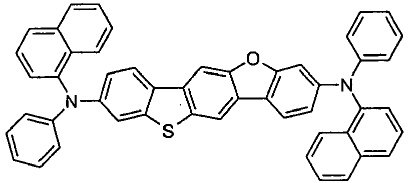 | 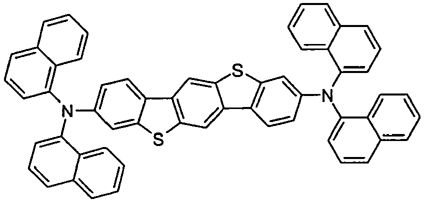 |
| (27) | (28) |
| 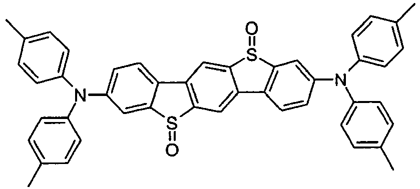 | 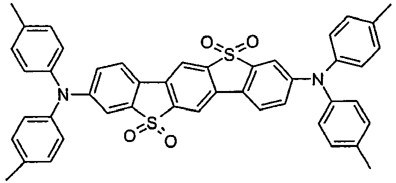 |
| (29) | (30) |
| 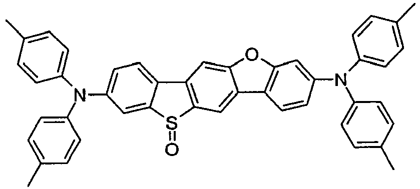 | 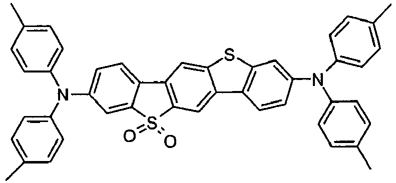 |
| (31) | (32) |
| 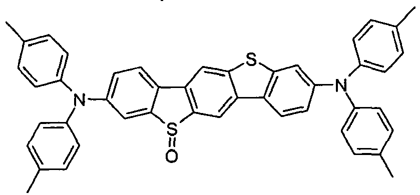 | 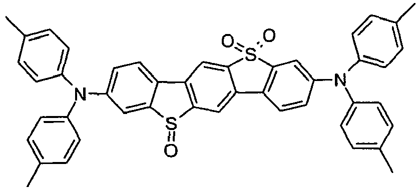 |
| (33) | (34) |
| 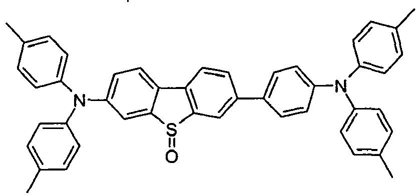 | 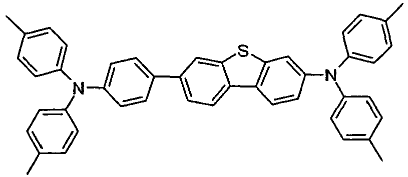 |
| (35) | (36) |
| 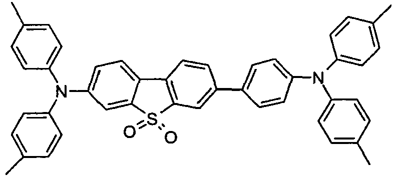 | 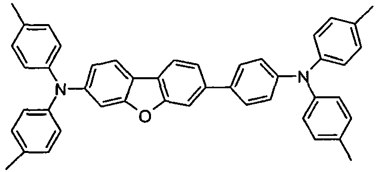 |
| (37) | (38) |
| 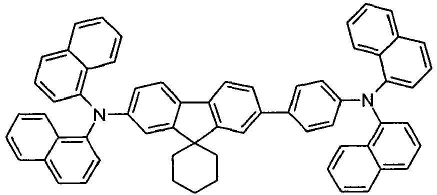 | 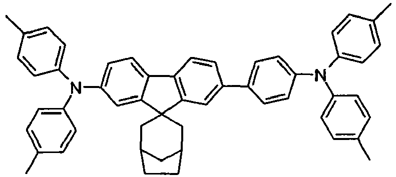 |
| (39) | (40) |
| 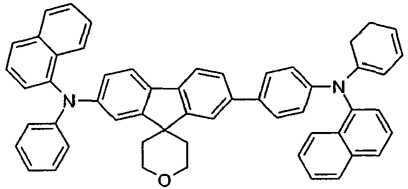 | 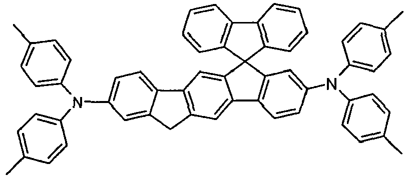 |
| (41) | (42) |
| 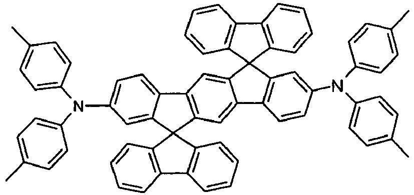 | 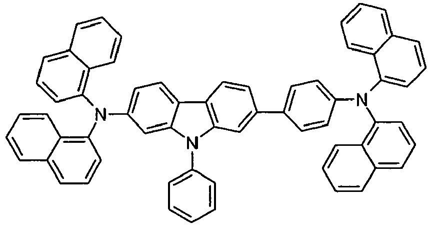 |
| (43) | (44) |
| 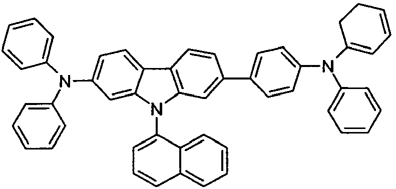 | 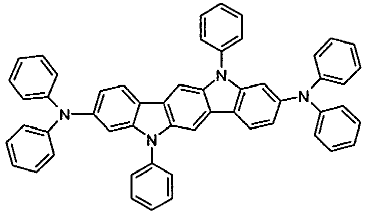 |
| (45) | (46) |
| 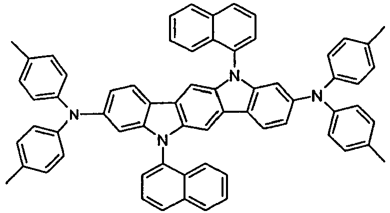 | 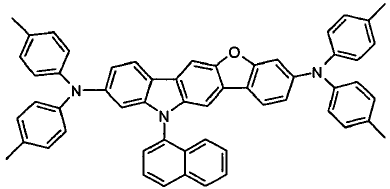 |
| (47) | (48) |
| 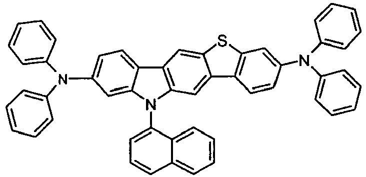 | 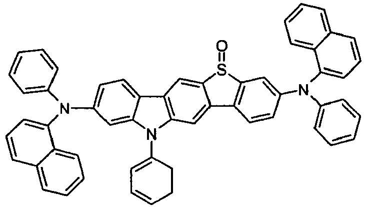 |
| (49) | (50) |
| 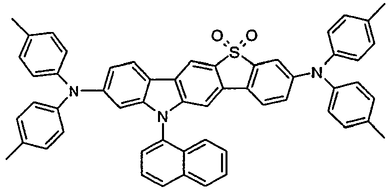 | 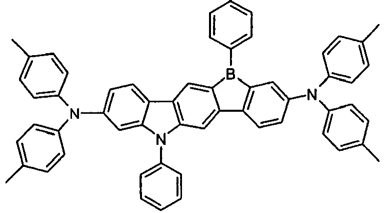 |
| (51) | (52) |
| 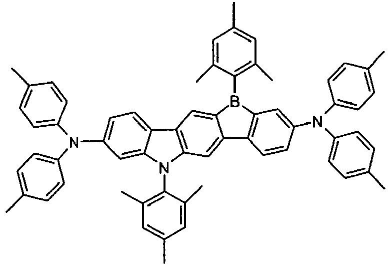 | 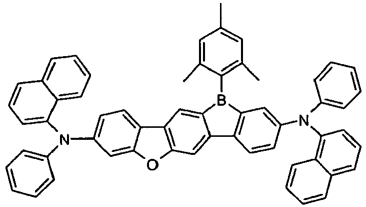 |
| (53) | (54) |
| 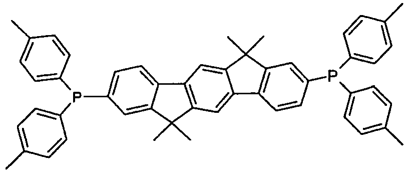 | 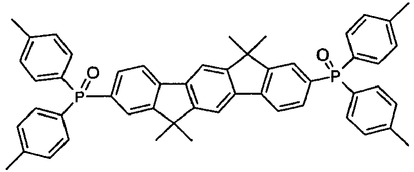 |
| (55) | (56) |
| 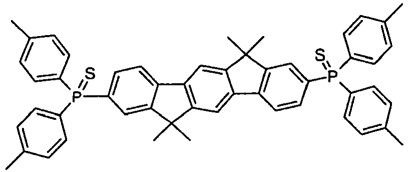 | 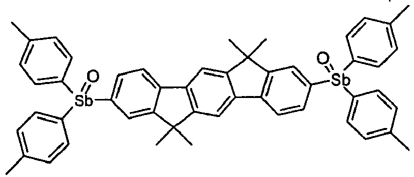 |
| (57) | (58) |
| 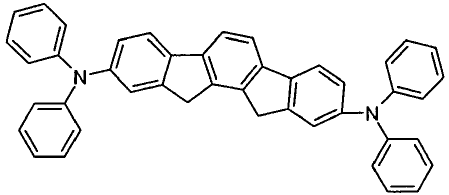 | 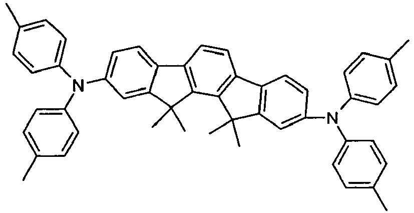 |
| (59) | (60) |
| 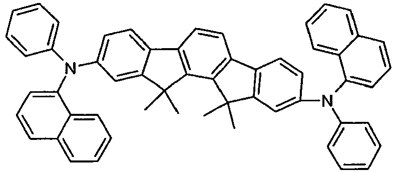 | 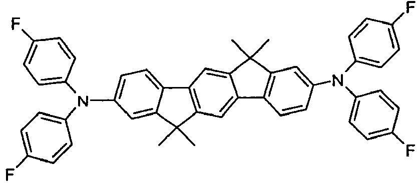 |
| (61) | (62) |
| 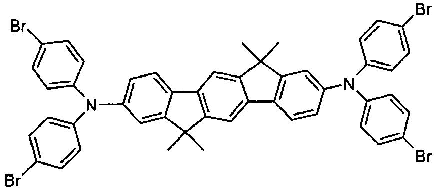 | 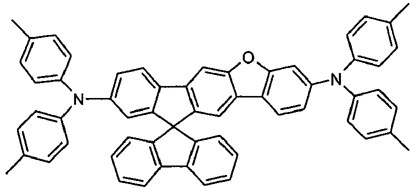 |
| (63) | (64) |
| 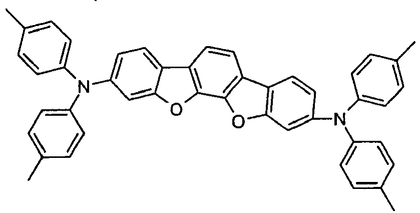 | 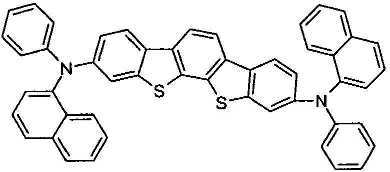 |
| (65) | (66) |
| 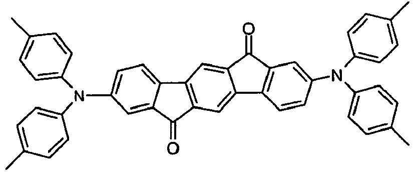 | 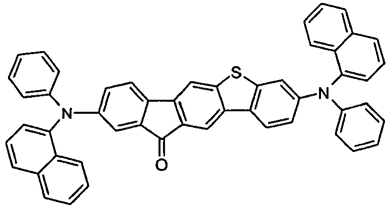 |
| (67) | (68) |
| 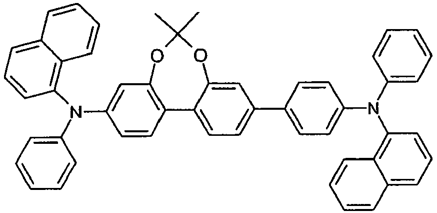 | 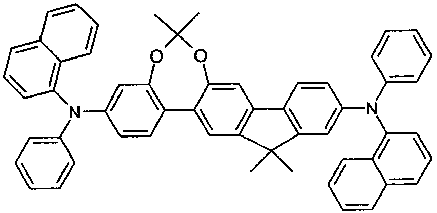 |
| (69) | (70) |
| 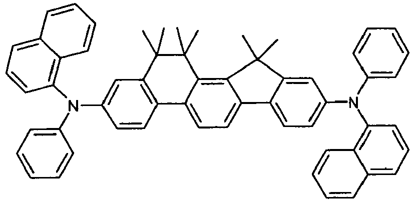 | 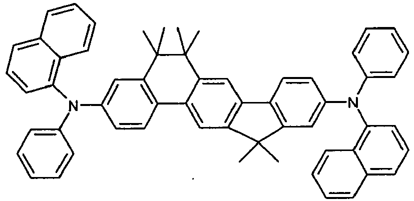 |
| (71) | (72) |
| 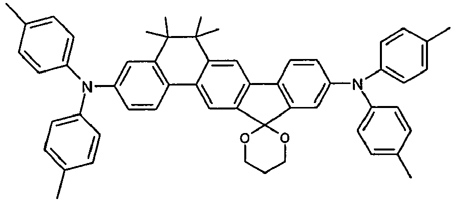 | 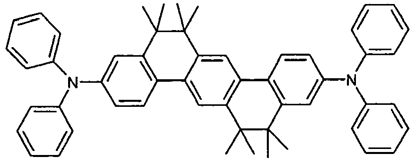 |
| (73) | (74) |
| 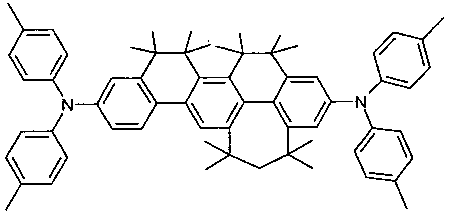 | 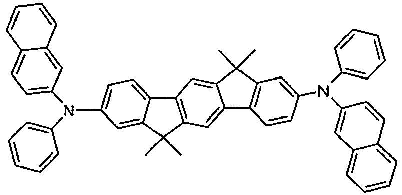 |
| (75) | (76) |
| 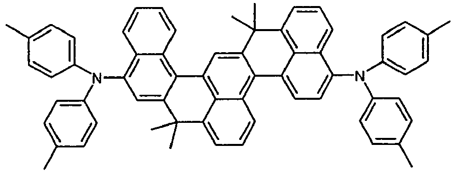 | 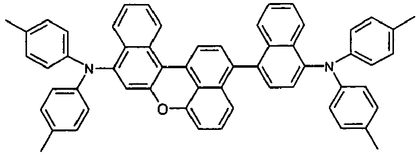 |
| (77) | (78) |
| 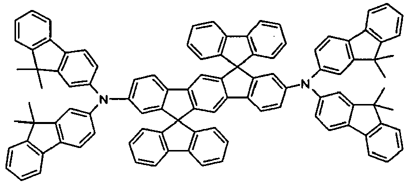 | 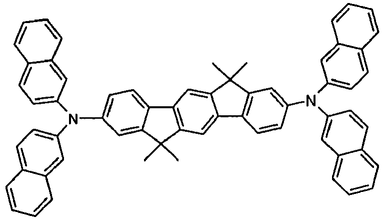 |
| (79) | (80) |
| 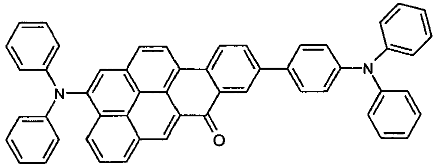 | 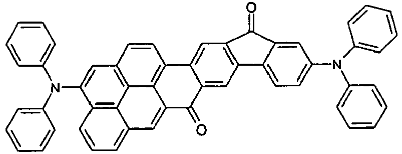 |
| (81) | (82) |
| 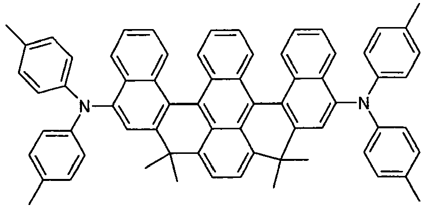 | 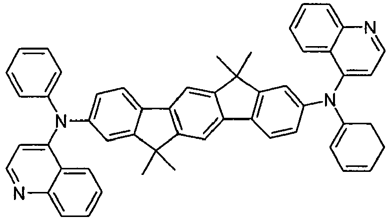 |
| (83) | (84) |
| 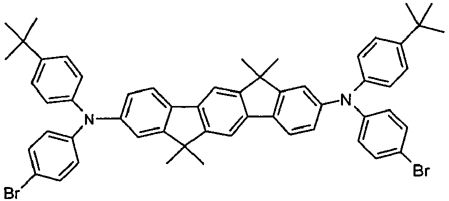 | 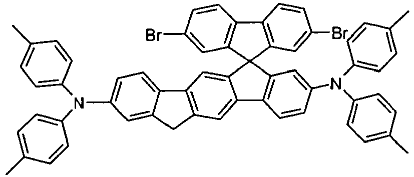 |
| (85) | (86) |
| 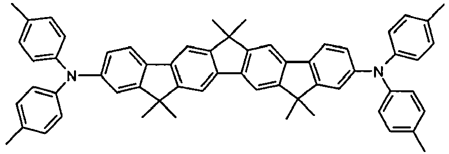 | 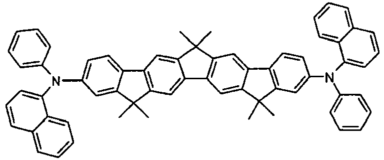 |
| (87) | (88) |
| 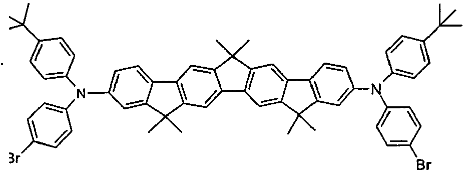 | 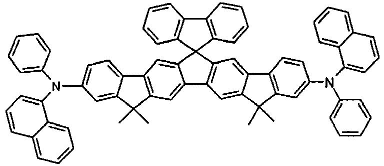 |
| (89) | (90) |
| 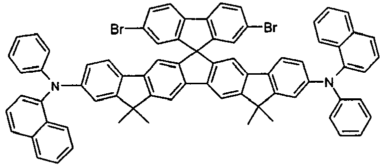 | 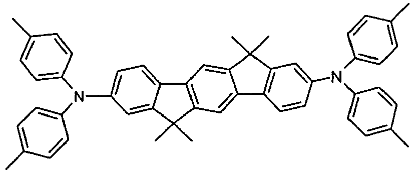 |
| (91) | (92) |
| 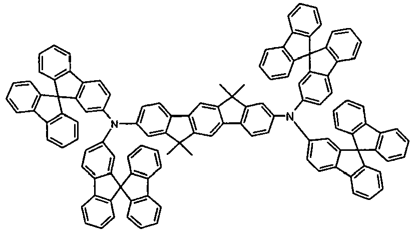 | 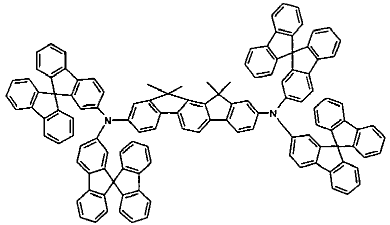 |
| (93) | (94) |
| 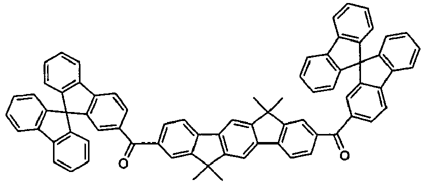 | 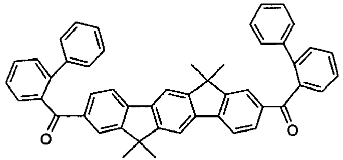 |
| (95) | (96) |
| 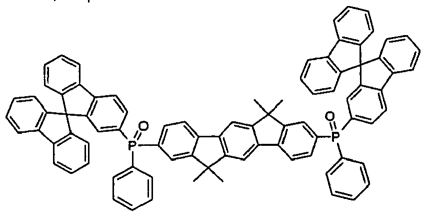 | 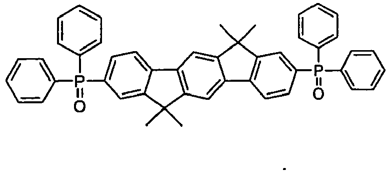 |
| (97) | (98) |
| 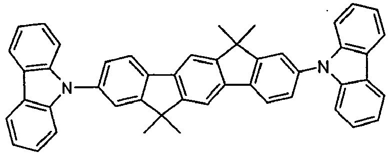 | 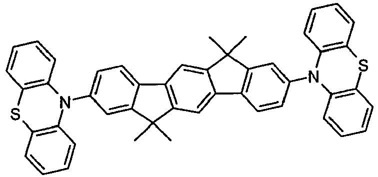 |
| (99) | (100) |
| 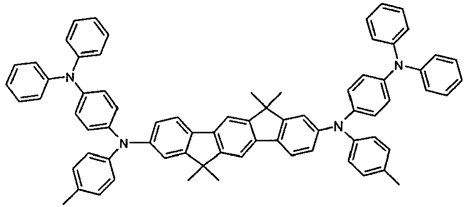 | 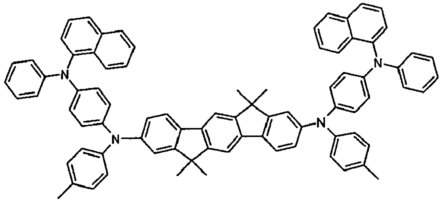 |
| (101) | (102) |
| 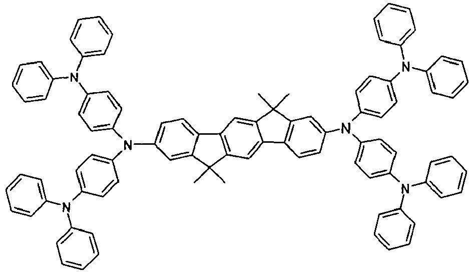 | 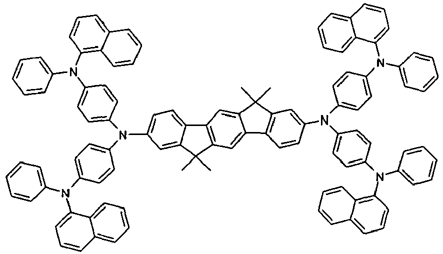 |
| (103) | (104) |

Die oben beschriebenen erfindungsgemäßen Verbindungen, z. B. Verbindungen gemäß den Strukturen (63), (85), (86), (89) und (91), können beispielsweise als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei bevorzugt über die Halogenfunktionalität.

Die erfindungsgemäßen Verbindungen können nach dem Fachmann bekannten Syntheseschritten, wie z. B. Bromierung, Suzuki-Kupplung, Hartwig-Buchwald-Kupplung, etc., dargestellt werden.

So können die Indenofluoren-Vorstufen beispielsweise hergestellt werden, wie in Syntheseschema 1 abgebildet: Durch Suzuki-Kupplung einer Benzolboronsäure und 1,4-Dibrom-2,5-bis(methylcarboxylat)benzol, gefolgt von Ringschluss unter Einwirkung einer starken Säure und Reduktion ist das unsubstituierte trans-Indenofluoren erhältlich, welches mit Alkylierungsmitteln alkyliert werden kann. Dieses kann entweder halogeniert, beispielsweise bromiert, werden oder durch Nitrierung und Reduktion in die entsprechende Aminoverbindung umgewandelt werden. Die Synthese von Bis-diarylamino-indenofluorenen kann durch Hartwig-Buchwald-Kupplung der Dibromverbindung erfolgen, wie in Syntheseschema 2 dargestellt.

Die Synthese von Indenofluoren enthaltenden Phosphinen bzw. Phosphinoxiden kann aus Dibromindenofluoren durch Lithiierung und Umsetzung mit Diarylchlorphosphinen erfolgen, wie in Syntheseschema 3 dargestellt. Oxidation ergibt dann das entsprechende Phosphinoxid. Ebenso lassen sich hier andere Elektrophile einsetzen, wie z. B. AsCl₃, AryIPCl₂, SOCl₂, Ar₂S₂, etc. Weitere erfindungsgemäße Verbindungen können nach diesen und ähnlichen Syntheseschemata leicht in dem Fachmann für organische Synthese bekannten Verfahren synthetisiert werden. Weiterhin können die erhaltenen Verbindungen nach Standardverfahren bromiert werden und können so als Monomere für Polymere, Oligomere oder Dendrimere eingesetzt werden. 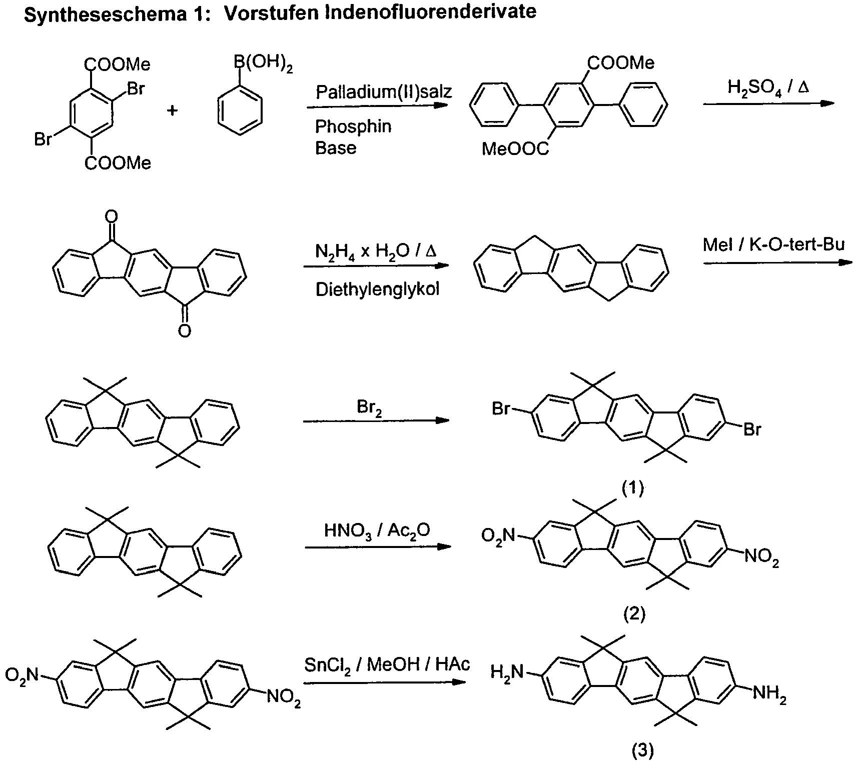 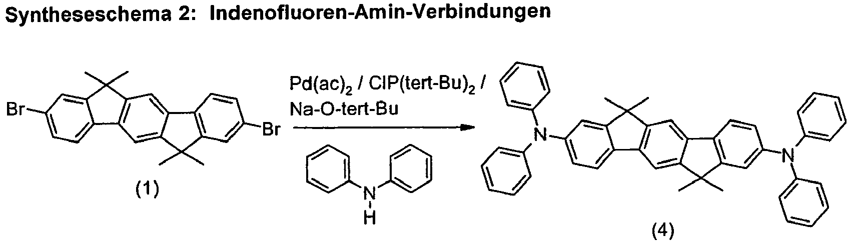 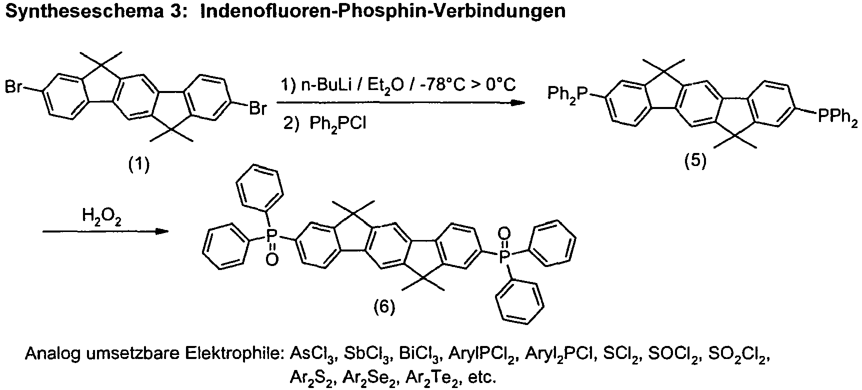 Die Verbindungen gemäß Formel (1) können in organischen Elektrolumineszenzvorrichtungen eingesetzt werden. Die genaue Verwendung der Verbindungen hängt dabei von den Substituenten und insbesondere von der Wahl der Gruppen Y und Z, aber auch von der Wahl der Gruppen X¹ bis X⁴ ab.

In einer bevorzugten Ausführungsform der Erfindung wird die Verbindung gemäß Formel (1) in der emittierenden Schicht eingesetzt, bevorzugt in Mischung mit mindestens einer weiteren Verbindung eingesetzt. Es ist bevorzugt, wenn die Verbindung gemäß Formel (1) in der Mischung die emittierende Verbindung (der Dotand) ist. Dies gilt insbesondere dann, wenn die Symbole Y und Z für Stickstoff stehen. Bevorzugte Hostmaterialien sind organische Verbindungen, deren Emission kurzwelliger ist als die der Verbindung gemäß Formel (1) oder die überhaupt nicht emittieren.

Weiterer Gegenstand der Erfindung sind daher Mischungen aus einer oder mehreren Verbindungen gemäß Formel (1) mit einem oder mehreren Hostmaterialien.

Der Anteil der Verbindung gemäß Formel (1) in der Mischung der emittierenden Schicht beträgt zwischen 0.1 und 99.0 Gew.%, bevorzugt zwischen 0.5 und 50.0 Gew.%, besonders bevorzugt zwischen 1.0 und 20.0 Gew.%, insbesondere zwischen 1.0 und 10.0 Gew.%. Entsprechend beträgt der Anteil des Hostmaterials in der Schicht zwischen 1.0 und 99.9 Gew.%, bevorzugt zwischen 50.0 und 99.5 Gew.%, besonders bevorzugt zwischen 80.0 und 99.0 Gew.%, insbesondere zwischen 90.0 und 99.0 Gew.%.

Als Hostmaterialien kommen verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 oder WO 05/084082), der Atropisomere (z. B. gemäß der nicht offen gelegten Anmeldung EP 04026402.0) oder der Boronsäurederivate (z. B. gemäß der nicht offen gelegten Anmeldung EP 05009643.7). Besonders bevorzugte Hostmaterialien sind ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide.

Es ist weiterhin insbesondere bevorzugt, wenn die Verbindungen gemäß Formel (1) als Lochtransportmaterial und/oder als Lochinjektionsmaterial eingesetzt werden. Dies gilt insbesondere dann, wenn die Symbole Y und Z und/oder die Symbole X¹ bis X⁴ für Stickstoff stehen. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert ist, beispielsweise mit F₄-TCNQ oder mit Verbindungen, wie sie in EP 1476881 oder EP 1596445 beschrieben werden.

Wird die Verbindung gemäß Formel (1) als Lochtransportmaterial in einer Lochtransportschicht eingesetzt, kann auch ein Anteil von 100 % bevorzugt sein, also die Verwendung dieser Verbindung als Reinmaterial.

Es ist weiterhin bevorzugt, die Verbindungen der Formel (1) als Elektronentransportmaterial und/oder als Lochblockiermaterial für fluoreszierende und phosphoreszierende OLEDs und/oder als Triplett-Matrixmaterial für phosphoreszierende OLEDs einzusetzen. Dies gilt insbesondere für Verbindungen, in denen die Gruppen Y und Z für C=O, P=O oder S=O stehen.

Auch in Polymeren können Verbindungen gemäß Formel (1) entweder als emittierende Einheit und/oder als lochtransportierende Einheit und/oder als elektronentransportierende Einheit eingesetzt werden.

Weiterhin bevorzugt sind organische Elektrolumineszenzvorrichtungen, dadurch gekennzeichnet, dass mehrere emittierende Verbindungen in derselben Schicht oder in unterschiedlichen Schichten verwendet werden, wobei mindestens eine dieser Verbindungen eine Struktur gemäß Formel (1) aufweist. Besonders bevorzugt weisen diese Verbindungen insgesamt mehrere Emissionsmaxima zwischen 380 nm und 750 nm auf, so dass insgesamt weiße Emission resultiert, d. h. außer der Verbindung gemäß Formel (1) wird noch mindestens eine weitere emittierende Verbindung verwendet, die fluoreszieren oder phosphoreszieren kann und die gelbes, orange oder rotes Licht emittiert. Insbesondere bevorzugt sind Dreischichtsysteme, wovon mindestens eine dieser Schichten eine Verbindung gemäß Formel (1) enthält und wobei die Schichten blaue, grüne und orange oder rote Emission zeigen (für den prinzipiellen Aufbau siehe z. B. WO 05/011013). Ebenso können für weiß emittierende OLEDs Breitbandemitter verwendet werden.

Außer Kathode, Anode und der emittierenden Schicht kann die organische Elektrolumineszenzvorrichtung noch weitere Schichten enthalten. Diese können beispielsweise sein: Lochinjektionsschicht, Lochtransportschicht, Lochblockierschicht, Elektronentransportschicht, Elektroneninjektionsschicht und/oder eine Charge-Generation Layer (T. Matsumoto et al., Multiphoton Organic EL Device Having Charge Generation Layer, IDMC 2003, Taiwan; Session 21 OLED (5)). Es sei aber an dieser Stelle darauf hingewiesen, dass nicht notwendigerweise jede dieser Schichten vorhanden sein muss. So werden insbesondere bei Verwendung von Verbindungen gemäß Formel (1) mit elektronenleitenden Hostmaterialien weiterhin sehr gute Ergebnisse erhalten, wenn die organische Elektrolumineszenzvorrichtung keine separate Elektronentransportschicht enthält und die emittierende Schicht direkt an die Elektroneninjektionsschicht oder an die Kathode grenzt. Alternativ kann das Hostmaterial auch gleichzeitig in einer Elektronentransportschicht als Elektronentransportmaterial dienen. Ebenfalls kann es bevorzugt sein, wenn die organische Elektrolumineszenzvorrichtung keine separate Lochtransportschicht enthält und die emittierende Schicht direkt an die Lochinjektionsschicht oder an die Anode grenzt. Weiterhin kann es bevorzugt sein, wenn die Verbindung gemäß Formel (1) gleichzeitig als Dotand in der emittierenden Schicht und als lochleitende Verbindung (als Reinsubstanz oder als Mischung) in einer Lochtransportschicht und/oder in einer Lochinjektionsschicht verwendet wird.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen. Diese Verfahren zur Herstellung von Schichten eignen sich insbesondere für Polymere.

Die erfindungsgemäßen Verbindungen weisen bei Verwendung in organischen Elektrolumineszenzvorrichtungen folgende überraschende Vorteile gegenüber dem Stand der Technik auf:
1. Die Effizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Bei Verwendung der erfindungsgemäßen Verbindungen als Lochtransportmaterial in einer Lochtransport- und/oder Lochinjektionsschicht zeigt sich, dass die Spannung unabhängig von der Schichtdicke der entsprechenden Lochtransport- bzw. Lochinjektionsschicht ist. Dagegen erhält man mit Materialien gemäß dem Stand der Technik bei dickeren Schichtdicken der Lochtransport- bzw. Lochinjektionsschichten einen deutlichen Spannungsanstieg, welcher wiederum zu einer geringeren Leistungseffizienz der OLED führt.
4. Die Verbindungen lassen sich gut und ohne erhebliche Zersetzung sublimieren, sind dadurch leichter zu verarbeiten und deshalb besser für die Verwendung in OLEDs geeignet als Materialien gemäß dem Stand der Technik. Ohne an eine bestimmte Theorie gebunden sein zu wollen, vermuten wir, dass die höhere thermische Stabilität auf die Abwesenheit olefinischer Doppelbindungen zurückzuführen ist.

Im vorliegenden Anmeldetext und auch in den im Weiteren folgenden Beispielen wird auf die Verwendung der erfindungsgemäßen Verbindungen in Bezug auf OLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Photorezeptoren oder auch organische Laserdioden (O-Laser), um nur einige Anwendungen zu nennen.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Di-tert-butyl-chlorphosphin, Amine, Anorganika, Lösemittel) bezogen werden. 6,12-Dihydro-[1,2b]indenofluoren wird nach Hadizad et al., Org. Lett. 2005, 7(5), 795-797, [1,2b]Indenofluoren-6,12-dion wird nach Deuschel et a/., Helv. Chim. Acta 1951, 34, 2403, 2-Brom-4,4'-di-tert-butylbiphenyl wird nach Tashiro et al., J. Org. Chem. 1979, 44(17), 3037, 1,4-Dibrom-2,5-diiodbenzol wird nach Chanteau et al., J. Org. Chem. 2003, 68(23), 8750, 3,9-Dibrom-5,11-dimethyl-indolo[3,2-b]carbazol wird analog zu 3,9-Dibrom-5,11-bisdodecyl-indolo[3,2-b]carbazol nach Li et al., Adv. Mat. 2005, 17(7), 849 dargestellt.

### Beispiel 1:2,8-Bis(diphenylamino)-6,6,12,12-tetramethyl-6,12-dihydro-indeno[1,2b]fluoren

### a) 6,6,12,12-Tetramethyl-6,12-dihydro-indeno[1,2b]fluoren

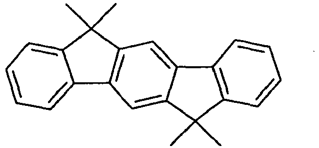

Die Darstellung erfolgt analog zur Darstellung von 9,9-Dimethylfluoren aus 6,12-Dihydro-indeno[1,2b]fluoren, Dimethylsulfat und Natronlauge nach JP 08113542. Ausbeute 86.0 % d. Th.; Reinheit 98 % n.¹H-NMR.

### b) 2,8-Dibrom-6,6,12,12-tetramethyl-6,12-dihydroindeno[1,2b]fluoren

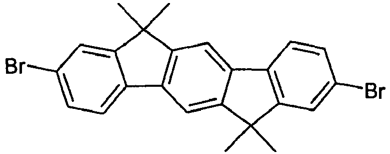

Eine Lösung von 122.0 g (393 mmol) 6,6,12,12-Tetramethyl-6,12-dihydro-indeno[1,2b]fluoren in 1800 ml Dichlormethan wird mit einer Lösung von 155.9 g (1260 mmol) Natriumcarbonat in 1000 ml Wasser versetzt. Unter Lichtausschluss und gutem Rühren gibt man bei +5 °C 56.4 ml (1100 mmol) Brom verdünnt mit 200 ml Dichlormethan tropfenweise zu, rührt 6 h nach, saugt vom ausgefallenen Niederschlag ab und wäscht diesen dreimal mit 300 ml Wasser: Ethanol (1:1, v:v) und dann dreimal mit 300 ml Ethanol. Ausbeute: 178.1 g (380 mmol), 96.8 % d. Th.; Reinheit: 99 % n. ¹H-NMR.

### c) 2,8-Bis(diphenylamino)-6,6,12,12-tetramethyl-6,12-dihydro-indeno[1,2b]fluoren

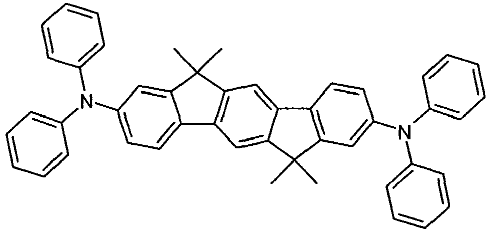

Eine Suspension von 46.8 g (100 mmol) 2,8-Dibrom-6,6,12,12-tetramethyl-6,12-dihydroindeno[1,2b]fluoren und 37.2 g (220 mmol) Diphenylamin in 1000 ml Toluol wird mit 23.1 g (240 mmol) Natrium-tert-butylat, 235 mg (1.3 mmol) Di-tert-butylchlorphosphin und 225 mg (1 mmol) Palladium(II)-acetat versetzt und anschließend 6 h unter Rückfluss erhitzt. Nach Erkalten gibt man 300 ml Wasser zu, filtriert vom Feststoff ab, wäscht diesen dreimal mit je 300 ml Wasser und dreimal mit je 300 ml Ethanol, kristallisiert anschließend fünfmal aus NMP um und sublimiert dann im Vakuum (p = 1 x 10⁻⁵ mbar, T = 360 °C). Ausbeute: 52.0 g (81 mmol), 80.6 % d. Th.; Reinheit: 99.9 % n. HPLC.

### Beispiel 2:2,8-Bis(bis(4-methylphenyl)amino)- 6,6,12,12-tetramethyl-6,12-dihydro-indeno[1,2b]fluoren

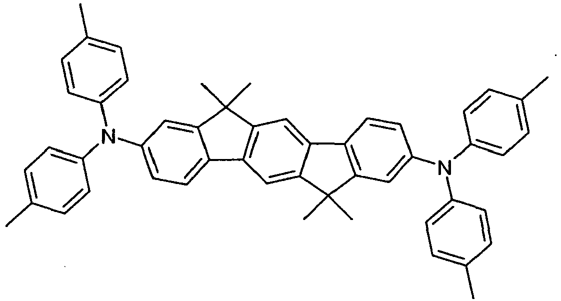

Durchführung analog Beispiel 1. Anstelle von Diphenylamin werden 43.4 g (220 mmol) Bis(4-methylphenyl)amin verwendet. Umkristallisation sechsmal aus o-Dichlorbenzol, Sublimation p = 1 x 10⁻⁵ mbar, T = 365 °C. Ausbeute: 45.1 g (64 mmol), 64.3 % d. Th.; Reinheit: 99.8 % n. HPLC.

### Beispiel 3: 2,8-Bis(bis(2-methylphenyl)amino)-6,6,12,12-tetramethyl-6,12-dihydro-indeno[1,2b]fluoren

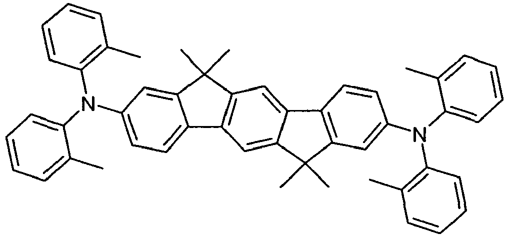

Durchführung analog Beispiel 1. Anstelle von Diphenylamin werden 43.4 g (220 mmol) Bis(2-methylphenyl)amin verwendet. Umkristallisation fünfmal aus o-Dichlorbenzol, Sublimation p = 1 x 10⁻⁵ mbar, T = 360 °C. Ausbeute: 57.4 g (82 mmol), 81.9 % d. Th.; Reinheit: 99.9 % n. HPLC.

### Beispiel 4: 2,8-Bis(bis(4-tert-butylphenyl)amino)-6,6,12,12-tetramethyl-6,12-dihydro-indeno[1,2b]fluoren

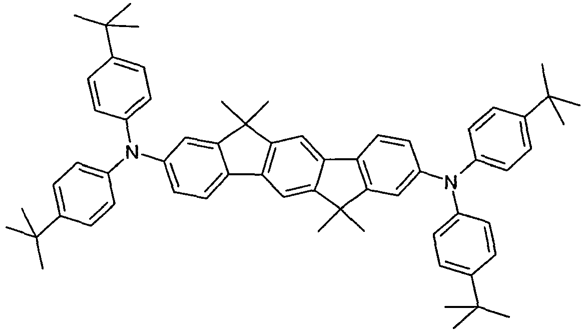

Durchführung analog Beispiel 1. Anstelle von Diphenylamin werden 61.9 g (220 mmol) Bis(4-tert-butylphenyl)amin verwendet. Umkristallisation fünfmal aus NMP, Sublimation p = 1 x 10⁻⁵ mbar, T = 350 °C. Ausbeute: 73.0 g (84 mmol), 84.0 % d. Th.; Reinheit: 99.9 % n. HPLC.

### Beispiel 5: Synthese weiterer Indenofluorenamine

Analog zu Beispiel 1 werden folgende Produkte mit einer Reinheit von 99.9 % n. HPLC dargestellt:

| Bsp. | Amin | Produkt |
|---|---|---|
| 6 | 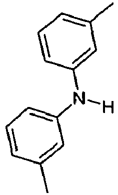 | 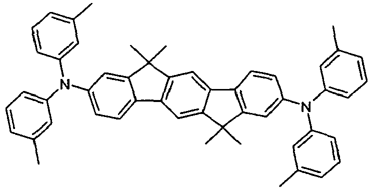 |
| 7 | 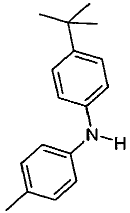 | 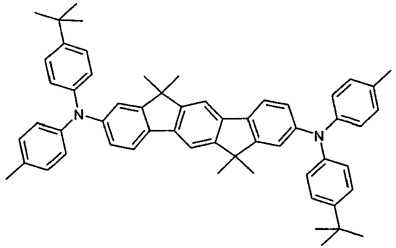 |
| 8 | 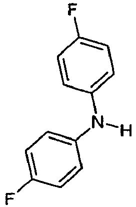 | 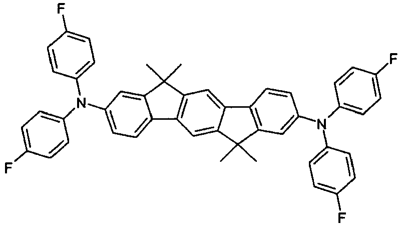 |
| 9 | 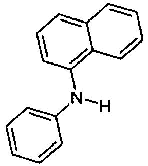 | 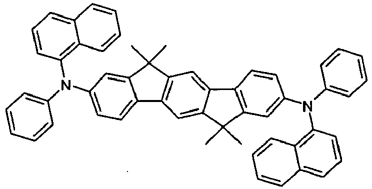 |
| 10 | 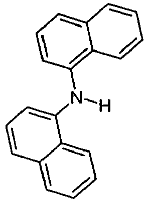 | 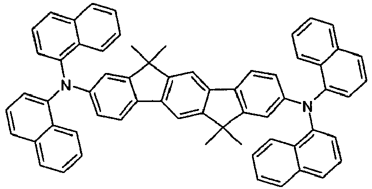 |
| 11 | 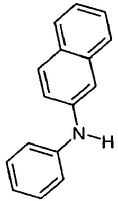 | 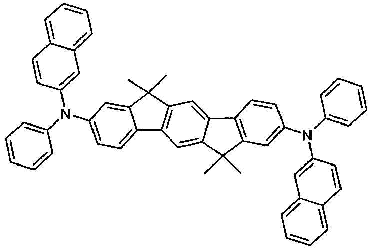 |
| 12 | 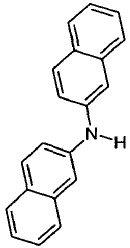 | 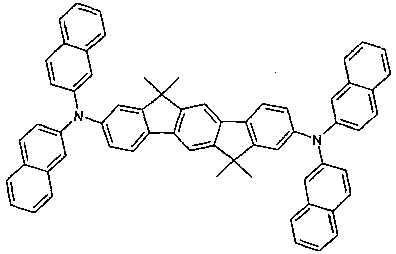 |
| 13 | 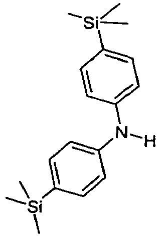 | 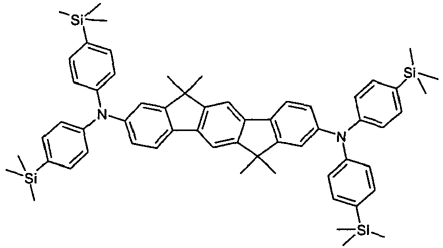 |
| 14 | 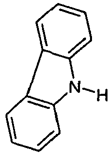 | 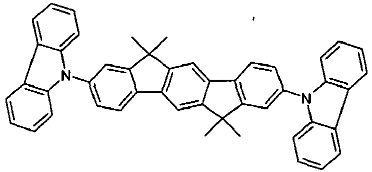 |
| 15 | 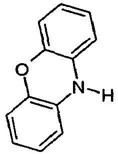 | 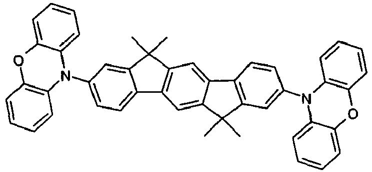 |
| 16 | 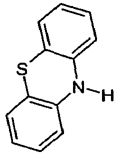 | 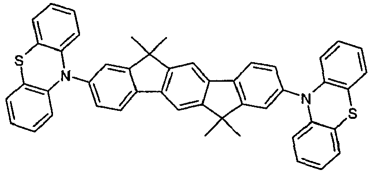 |

In Analogie zu diesen Synthesen lassen sich auch die entsprechenden cis-Indenofluoren-Derivate synthetisieren, wobei das cis-Indenofluoren-Dibromid als Ausgangsverbindung gemäß WO 04/113412 synthetisiert werden kann.

### Beispiel 17: 2,8-Bis(bis(4-tert-butylphenyl)amino)-di-spiro-[2,7-di-tert-butyl-fluoren-9,6'-indenofluoren-[1,2b]-fluoren-12',9"-fluoren]

### a) Dispiro-[2,7-di-tert-butyl-fluoren-9,6'-indenofluoren-[1,2b]-fluoren-12',9"-fluoren]

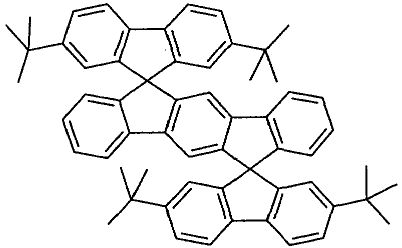

Aus 6.2 g (255 mmol) Magnesium und 86.3 g (250 mmol) 2-Brom-4,4'-di-tert-butylbiphenyl in 500 ml THF wird das entsprechende Grignard-Reagenz dargestellt. Zu diesem Grignard-Reagenz gibt man weitere 500 ml THF und 28.8 g (100 mmol) [1,2b]Indenofluoren-6,12-dion. Die Reaktionsmischung wird 10 h unter Rückfluss erhitzt, nach Erkalten mit 50 ml Ethanol versetzt und im Vakuum zur Trockene eingeengt. Der Rückstand wird in einem Gemisch aus 1000 ml Essigsäure und 25 ml konz. Salzsäure 3 h unter Rückfluss erhitzt. Nach Erkalten werden die farblosen Kristalle abgesaugt, mit 100 ml Essigsäure, dann dreimal mit je 100 ml Ethanol gewaschen und im Vakuum getrocknet. Anschließend wird zweimal aus NMP umkristallisiert. Ausbeute: 56.9 g (73 mmol), 73.0 % d. Th.; Reinheit: 99 % n.¹H-NMR.

### b) 2,8-Dibrom-dispiro-[2,7-di-tert-butyl-fluoren-9,6'-indenofluoren-[1,2b]-fluoren-12',9"-fluoren]

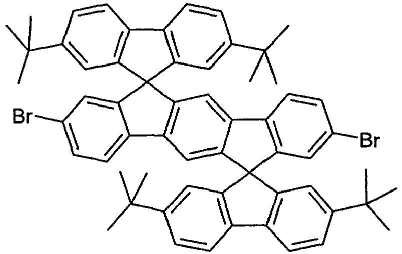

Eine Lösung von 39.0 g (50 mmol) Dispiro-[2,7-di-tert-butyl-fluoren-9,6'-indenofluoren-[1,2b]-fluoren-12',9"-fluoren] in 2000 ml Dichlormethan wird mit einer Lösung von 16.8 g (200 mmol) Natriumhydrogencarbonat in 500 ml Wasser versetzt. Die zweiphasige Mischung wird unter gutem Rühren tropfenweise mit 5.4 ml (105 mmol) Brom versetzt und 16 h nachgerührt. Nach Zugabe von 1000 ml Ethanol wird vom Feststoff abgesaugt, dieser wird fünfmal mit je 300 ml Wasser und dreimal mit je 200 ml Ethanol gewaschen, im Vakuum getrocknet und aus o-Dichlorbenzol umkristallisiert. Ausbeute: 38.7 g (41 mmol), 82.6 % d. Th.; Reinheit: 99 % n.¹H-NMR.

### c) 2,8-Bis(bis(4-tert-butylphenyl)amino)-dispiro-[2,7-di-tert-butyl-fluoren-9,6'-indenofluoren-[1,2b]-fluoren-12',9"-fluoren]

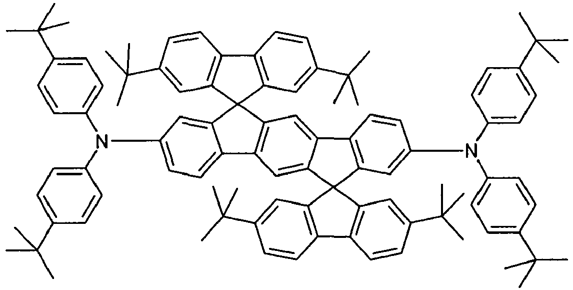

Durchführung analog Beispiel 1. Anstelle von 2,8-Dibrom-6,6,12,12-tetramethyl-6,12-dihydro-indeno[1,2b]fluoren werden 28.1 g (30 mmol) 2,8-Dibrom-dispiro-[2,7-di-tert-butyl-fluoren-9,6'-indenofluoren-[1,2b]-fluoren-12',9"-fluoren], anstelle von Diphenylamin werden 18.6 g (66 mmol) Di-(4-tert-butylphenyl)amin verwendet. Umkristallisation fünfmal aus o-Dichlorbenzol, Sublimation p = 1 x 10⁻⁵ mbar, T = 390 °C. Ausbeute: 23.2 g (17 mmol), 57.8 % d. Th.; Reinheit: 99.9 % n. HPLC.

### Beispiel 18: 2,8-Bis(bis(4-methylphenyl)amino)-di-spiro-[fluoren-9,6'-indenofluoren-[1,2b]-fluoren-12',9"-fluoren]

### a) 2,8-Dibrom-[1,2b]Indenofluoren-6,12-dion

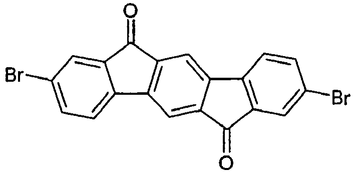

Eine Suspension von 56.5 g (200 mmol) [1,2b]Indenofluoren-6,12-dion und 3.0 g Eisen(III)chlorid (wasserfrei) in 2000 ml 1,2-Dichlorethan wird bei 80 °C tropfenweise mit 30.7 ml (600 mmol) Brom versetzt und 30 h bei 80 °C gerührt. Nach Erkalten wird vom ausgefallenen Feststoff abgesaugt, dieser wird zweimal mit je 1000 ml Ethanol am Rückfluss ausgerührt und im Vakuum getrocknet. Ausbeute: 81.6 g (185 mmol), 92.7 % d. Th.; Reinheit: 95 % n. ¹H-NMR.

### b) 2,8-Dibrom-dispiro-[fluoren-9,6'-indenofluoren-[1,2b]-fluoren-12',9"-fluoren]

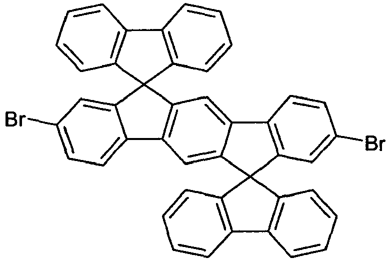

Darstellung analog Beispiel 17a. Anstelle von 2-Brom-4,4'-di-tert-butyl-biphenyl und [1,2b]Indenofluoren-6,12-dion werden 58.3 g (250 mmol) 2-Brom-biphenyl und 44.0 g (100 mmol) 2,8-Dibrom-[1,2b]indenofluoren-6,12-dion eingesetzt. Umkristallisation aus o-Dichlorbenzol. Ausbeute: 24.5 g (34 mmol), 34.4 % d. Th.; Reinheit: 98 % n. ¹H-NMR.

### c) 2,8-Bis(diphenylamino)-dispiro-[fluoren-9,6'-indenofluoren-[1,2b]-fluoren-12',9"-fluoren]

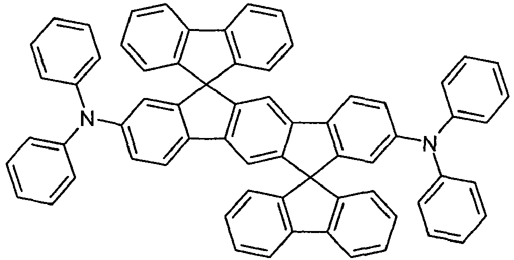

Darstellung analog Beispiel 1c. Anstelle von 2,8-Dibrom-6,6,12,12-tetramethyl-6,12-dihydro-indeno[1,2b]fluoren werden 71.3 g (100 mmol) 2,8-Dibrom-dispiro-[fluoren-9,6'-indenofluoren-[1,2b]-fluoren-12',9"-fluoren] eingesetzt. Umkristallisation aus o-Dichlorbenzol, Sublimation bei p = 1 x 10⁻⁵ mbar, T = 390 °C. Ausbeute: 71.9 g (81 mmol), 80.9 % d. Th.; Reinheit: 99.7 % n. HPLC.

### Beispiel 19: 2,8-Bis(phenylcarbonyl)-(6,6,12,12-tetramethyl-6,12-dihydroindeno[1,2b]fluoren)

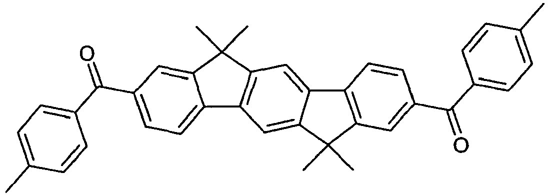

Eine auf -78 °C gekühlte Suspension von 46.8 g (100 mmol) 2,8-Dibrom-6,6,12,12-tetramethyl-6,12-dihydroindeno[1,2b]fluoren in 700 ml THF wird tropfenweise mit 84.0 ml (210 mmol) n-Buthyllithium (2.5 M in Hexan) versetzt. Man lässt die Mischung langsam während 2 h auf 0 °C erwärmen, rührt 1 h bei 0 °C nach, versetzt dann mit einer Mischung von 27.5 ml (230 mmol) 4-Methylbenzonitril in 100 ml THF und rührt bei Raumtemperatur 16 h nach. Man versetzt die Mischung tropfenweise mit 20 ml Ethanol, dann mit 100 ml 1 N Salzsäure und erhitzt 5 h unter Rückfluss. Nach Erkalten wird das Lösemittel im Vakuum entfernt, der Rückstand wird in 500 ml NMP, 20 ml Wasser und 5 ml Essigsäure aufgenommen und 5 h unter Rückfluss erhitzt. Nach Erkalten werden die Kristalle abgesaugt und dreimal aus NMP umkristallisiert. Sublimation bei p = 1 x 10⁻⁵ mbar, T = 320 °C. Ausbeute: 44.2 g (81 mmol), 80.8 % d. Th.; Reinheit: 99.9 % n. HPLC.

### Beispiel 20: Synthese weiterer Indenofluoren-Carbonyle

Analog zu Beispiel 19 werden folgende Produkte mit einer Reinheit von 99.9 % n. HPLC dargestellt.

| Bsp. | Nitril | Produkt |
|---|---|---|
| 21 | 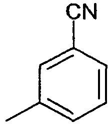 | 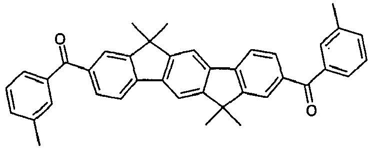 |
| 22 | 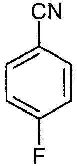 | 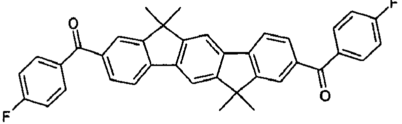 |
| 23 | 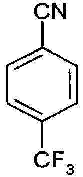 | 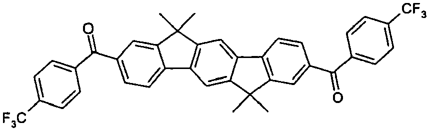 |
| 24 | 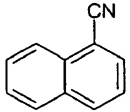 | 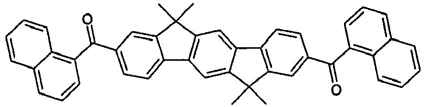 |
| 25 | 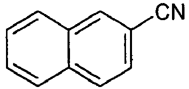 | 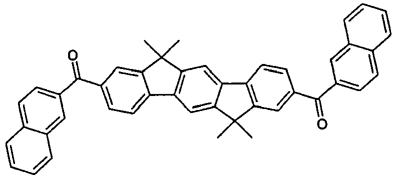 |
| 26 | 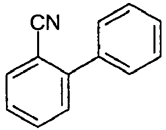 | 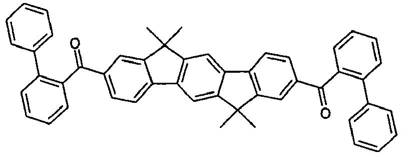 |
| 27 | 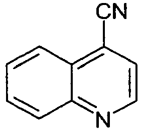 | 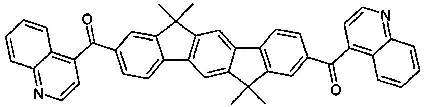 |
| 28 | 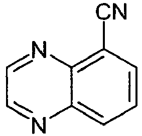 | 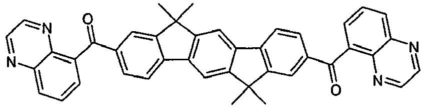 |
| 29 | 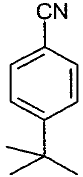 | 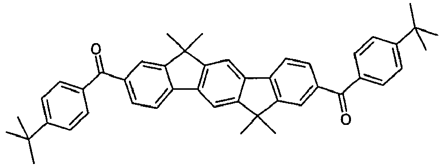 |
| 30 | 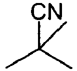 | 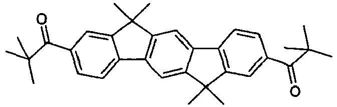 |

### Beispiel 31: 2,8-Bis(diphenylphosphinyl)-(6,6,12,12-tetramethyl-6,12-dihydroindeno[1,2b]fluoren)

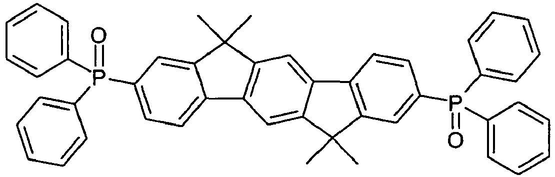

Eine auf -78 °C gekühlte Suspension von 46.8 g (100 mmol) 2,8-Dibrom-6,6,12,12-tetramethyl-6,12-dihydroindeno[1,2b]fluoren in 700 ml THF wird mit 84.0 ml (210 mmol) n-Buthyllithium (2.5 M in n-Hexan) versetzt. Man lässt die Mischung langsam während 2 h auf 0 °C erwärmen, rührt 1 h bei 0 °C nach, versetzt dann mit einer Mischung von 41.3 ml (230 mmol) Chlordiphenylphosphin in 100 ml THF und rührt bei Raumtemperatur 16 h nach. Nach Zugabe von 10 ml Ethanol wird das Lösemittel im vollständig Vakuum abgezogen, der Rückstand wird in 500 ml Essigsäureethylester gelöst, die organische Phase wird dreimal mit 300 ml Wasser gewaschen, dann tropfenweise unter gutem Rühren mit einem Gemisch aus 22.2 ml (250 mmol) Wasserstoffperoxid und 100 ml Wasser versetzt und 16 h bei Raumtemperatur gerührt. Der ausgefallene Feststoff wird abgesaugt, mit Ethanol gewaschen, getrocknet und aus Chlorbenzol umkristallisiert. Sublimation bei p = 1 x 10⁻⁵ mbar, T = 340 °C. Ausbeute: 46.0 g (65 mmol), 64.7 % d. Th.; Reinheit: 99.9 % n. HPLC.

### Beispiel 32: Synthese weiterer Indenofluoren-Phosphinoxide

Analog zu Beispiel 31 werden folgende Produkte mit einer Reinheit von 99.9 % n. HPLC dargestellt:

| Bsp. | Chlorphosphin | Produkt |
|---|---|---|
| 33 | 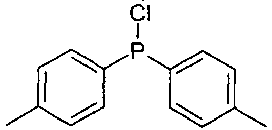 | 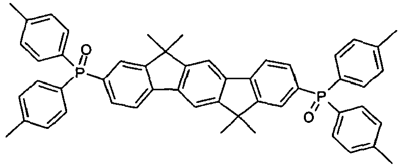 |
| 34 | 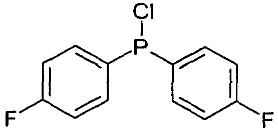 | 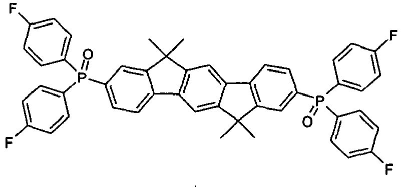 |
| 35 | 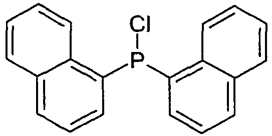 | 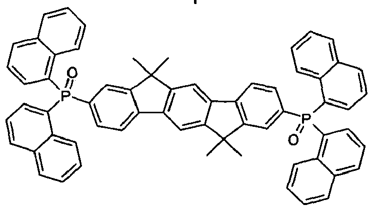 |
| 36 | 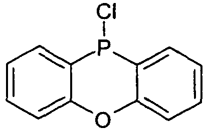 | 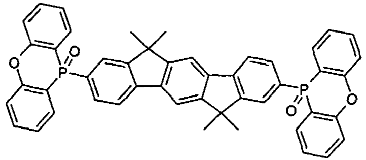 |

### Beispiel 37: 3,9-Bis(diphenylamino)-5, 11-di-methylindolo-[3,2-b]carbazol

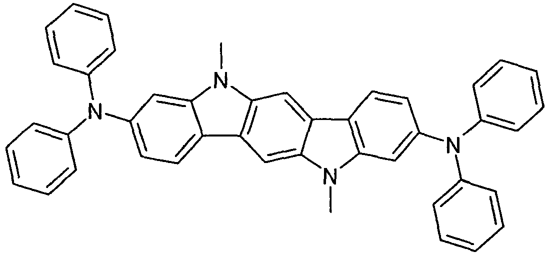

Darstellung analog Beispiel 1c. Anstelle von 2,8-Dibrom-6,6,12,12-tetramethyl-6,12-dihydroindeno[1,2b]fluoren werden 44.2 g (100 mmol) 3,9-Dibrom-5,11-dimethylindolo[2,3-b]carbazol verwendet. Umkristallisation aus NMP. Sublimation p = 1 x 10⁻⁵ mbar, T = 350 °C. Ausbeute: 43.9 g (71 mmol), 70.9 % d. Th.; Reinheit: 99.8 % n. HPLC.

### Beispiel 38: Synthese weiterer Indolocarbazol-Derivate

Analog zu Beispiel 37 werden folgende Produkte mit einer Reinheit von 99.9 % n. HPLC dargestellt:

| Bsp. | Amin | Produkt |
|---|---|---|
| 39 | 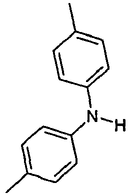 | 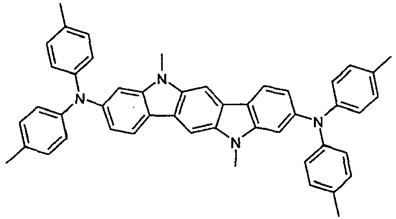 |
| 40 | 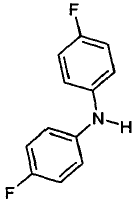 | 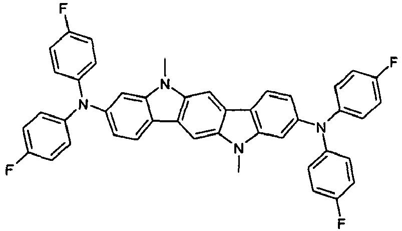 |
| 41 | 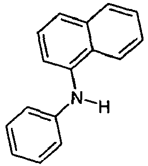 | 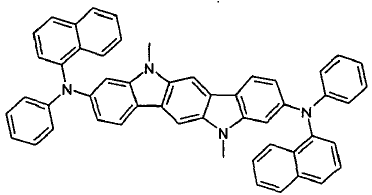 |
| 42 | 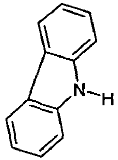 | 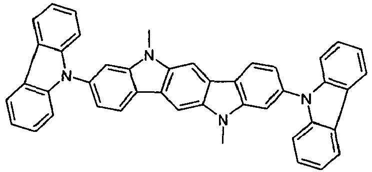 |
| 43 | 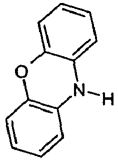 | 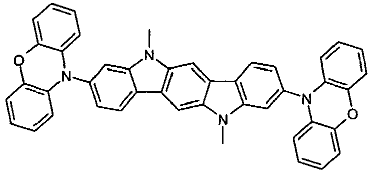 |
| 44 | 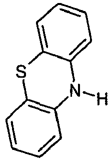 | 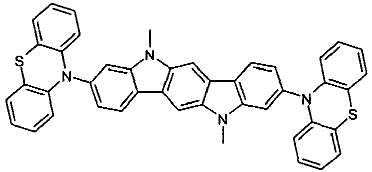 |

### Beispiel 45: 3,9-Bis(diphenylamino)-5,11-di-phenylphosphindolo-[3,2-b]dibenzophosphol

### a) 2',5'-Dibrom-4,4,4',4'-tetra-p-tolyl-[1,1',4,4]terphenyl-4,4'-diamin

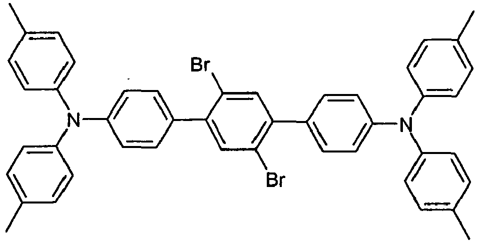

Eine Suspension von 24.4 g (50 mmol) 1,4-Dibrom-2,5-düodbenzol, 51.9 g (130 mmol) 4-[4,4,5,5-Tetramethyl-[1,3,2]dioxaborolan-2-yl]-di-p-tolylamin, 26.5 g (250 mmol) Natriumcarbonat und 116 mg (0.1 mmol) Tetrakis-(triphenylphosphino)palladium(0) in einem Gemisch aus 300 ml Toluol, 100 ml Dioxan und 300 ml Wasser wird 18 h unter Rückfluss erhitzt. Nach Erkalten wird die Reaktionsmischung mit 500 ml Ethanol versetzt, der Feststoff wird abgesaugt, dreimal mit je 200 ml Wasser und dreimal mit je 200 ml Ethanol gewaschen, im Vakuum getrocknet und aus Dioxan umkristallisiert. Ausbeute: 18.6 g (24 mmol), 47.7 % d. Th.; Reinheit: 97 % n. NMR.

### b) 3,9-Bis(diphenylamino)-5, 11-di-phenylphosphindolo-[3,2-b]dibenzophosphol-5,11-oxid

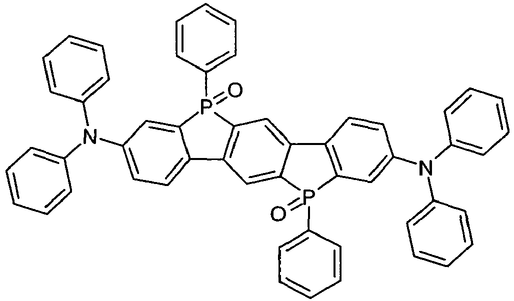

Eine auf -78 °C gekühlte Lösung von 15.6 g (20 mmol) 2',5'-Dibrom-4,4,4',4'-tetra-p-tolyl-[1,1',4,4]terpenyl-4,4'-diamin in 500 ml THF wird tropfenweise mit 16.8 ml (42 mmol) n-Buthyllithium (2.5 M in n-Hexan) versetzt, 3 h bei -78 °C nachgerührt und mit einem Gemisch aus 6.2 ml (44 mmol) Phenylphosphonigsäuredichlorid und 50 ml THF während 1 min. versetzt. Nach langsamem Erwärmen auf Raumtemperatur wird das Lösemittel im Vakuum komplett entfernt, der Rückstand wird in 200 ml 1,2-Dichlorethan aufgenommen, mit 26.7 g (200 mmol) wasserfreiem Aluminiumchlorid versetzt und 15 h unter Rückfluss erhitzt. Nach Erkalten gibt man 200 ml 5 N Salzsäure zu, trennt die organische Phase ab, wäscht diese einmal mit 100 ml 5 N Salzsäure und fünfmal mit je 300 ml Wasser, trocknet über Magnesiumsulfat, entfernt das Lösemittel im Vakuum und kristallisiert aus NMP um. Sublimation p = 1 x 10⁻⁵ mbar, T = 360 °C. Ausbeute: 6.2 g (7.7 mmol), 38.3 % d. Th.; Reinheit: 99.8 % n. HPLC, alle Stereoisomere umfassend.

### Beispiel 46: Herstellung von OLEDs mit Indenofluoren-Diaminen als Lochinjektionsmaterial bzw. Lochtransportmaterial in fluoreszierenden OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 47-62 werden die Ergebnisse verschiedener OLEDs vorgestellt. Der grundlegende Aufbau und die verwendeten Materialien (außer der Lochtransportschicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM1) | **B2** (Verbindung nach Beispiel 2) |
| oder | **B1** (Verbindung nach Beispiel 1) |
| oder | **B9** (Verbindung nach Beispiel 9) |
| oder | **B15** (Verbindung nach Beispiel 15) |
| oder | **B37** (Verbindung nach Beispiel 37) |
| oder: | als Vergleichsbeispiel 4,4',4"-Tris(N-1-naphthyl-N-phenylamino)-triphenylamin (abgekürzt als NaphDATA bezogen von SynTec) |
| Lochtransportschicht (HTM2) | 20 nm NPB (N-Naphthyl-N-phenyl-4,4'-diaminobiphenyl) |
| Emissionschicht (EML) | 30 nm, dotierte Schicht aus 9,10-Bis(1-naphthylanthracen) als Hostmaterial (abgekürzt **H1**), dotiert mit 5 % Tris[4-(2,2-diphenyl-vinyl)-phenyl]amin als Dotand (abgekürzt **D1**, aufgedampft, synthetisiert nach WO 06/000388) |
| Elektronenleiter (ETL) | 20 nm AlQ₃ (bezogen von SynTec, Tris(chinolinato)aluminium(III)) |
| Kathode | 1 nm LiF, darauf 150 nm Al. |

Die OLED können ebenso ohne PEDOT als Lochinjektionsschicht hergestellt werden. In diesen Fällen ist dann das erfindungsgemäße Indenofluoren-Diamin-Derivat die Lochinjektionsverbindung. Diese OLEDs zeigen vergleichbar gute Eigenschaften.

Diese OLEDs werden standardmäßig charakterisiert; hierfür werden die Elektrolumineszenzspektren, die Effizienz (gemessen in cd/A) und die Leistungseffizienz (gemessen in lm/W) in Abhängigkeit der Helligkeit, berechnet aus Strom-Spannungs-Helligkeit-Kennlinien (IUL-Kennlinien), bestimmt.

In Tabelle 1 sind die Ergebnisse einiger OLEDs (Beispiele 47 bis 62), bei denen die Schichtdicke der Lochtransportschicht (HTM1) variiert wird, zusammengefasst. Als Vergleichsmaterial wird in den Vergleichsbeispielen NaphDATA verwendet.

Das Hostmaterial **H1** ist 9,10-Bis(1-naphthyl)anthracen, als Dotand wird **D1** eingesetzt. Beide sind im Folgenden dargestellt:

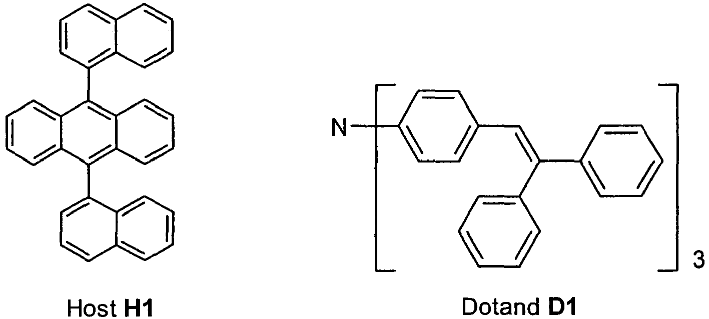

Wie man den erfindungsgemäßen Beispielen 51 bis 62 in der Tabelle 1 entnehmen kann, zeigen OLEDs enthaltend das erfindungsgemäße Lochtransportmaterial (HTM1) eine deutlich niedrigere Betriebsspannung als mit NaphDATA gemäß dem Stand der Technik als Lochtransportmaterial. Weiterhin ist die Betriebsspannung unabhängig von der Schichtdicke der Lochtransportschicht. Diese Eigenschaft ist für den Aufbau vollfarbiger Displays von großem Vorteil, da man durch Variation der Schichtdicke der Lochtransportschicht die Dicke der Pixel der Grundfarben Blau, Grün und Rot gleich gestalten kann. Das erfindungsgemäße Lochtransportmaterial kann hier also als Dickenausgleichsschicht dienen, ohne dass dadurch die elektooptischen Eigenschaften des Devices nachteilig beeinflusst werden. Wie den Vergleichsbeispielen zu entnehmen ist, ist dies für Lochstransportmaterial (NaphDATA) gemäß dem Stand der Technik nicht der Fall: Hier wird bei größerer Schichtdicke der Lochtransportschicht eine deutlich höhere Betriebsspannung benötigt.

**Tabelle 1**

| **Beispiel** | **HTL 1 or HIL** | **HTL 2** | **Max. Effizienz (cd/A)** | **Spannung (V) bei 1000cd/m²** | **CIE** |
|---|---|---|---|---|---|
| Beispiel 47 (Vergleich) | NaphDATA (20 nm) | NPB (20 nm) | 7.5 | 6.1 | x=0.16 |
| | | | | | y=0.25 |
| Beispiel 48 (Vergleich) | NaphDATA (50 nm) | NPB (20 nm) | 7.2 | 6.0 | x=0.16 |
| | | | | | y=0.25 |
| Beispiel 49 (Vergleich) | NaphDATA (100 nm) | NPB (20 nm) | 6.4 | 7.9 | x=0.16 |
| | | | | | y=0.24 |
| Beispiel 50 (Vergleich) | NaphDATA (150 nm) | NPB (20 nm) | 5.7 | 8.4 | x=0.16 |
| | | | | | y=0.26 |
| Beispiel 51 | **B2 (20 nm)** | NPB (20 nm) | 8.5 | 5.2 | x=0.16 |
| | | | | | y=0.25 |
| Beispiel 52 | **B2 (50 nm)** | NPB (20 nm) | 8.6 | 5.3 | x=0.16 |
| | | | | | y=0.25 |
| Beispiel 53 | **B2 (100 nm)** | NPB (20 nm) | 8.6 | 5.5 | x=0.16 |
| | | | | | y=0.24 |
| Beispiel 54 | **B2 (150 nm)** | NPB (20 nm) | 8.7 | 5.6 | x=0.16 |
| | | | | | y=0.26 |
| Beispiel 55 | **B1c (20 nm)** | NPB (20 nm) | 8.2 | 5.4 | x=0.16 |
| | | | | | y=0.25 |
| Beispiel 56 | **B1c (100 nm)** | NPB (20 nm) | 8.3 | 5.5 | x=0.16 |
| | | | | | y=0.24 |
| Beispiel 57 | **B9 (20 nm)** | NPB (20 nm) | 8.2 | 6.1 | x=0.16 |
| | | | | | y=0.25 |
| Beispiel 58 | **B9 (100 nm)** | NPB (20 nm) | 8.1 | 6.3 | x=0.16 |
| | | | | | y=0.24 |
| Beispiel 59 | **B15 (20 nm)** | NPB (20 nm) | 8.8 | 5.6 | x=0.16 |
| | | | | | y=0.25 |
| Beispiel 60 | **B15 (100 nm)** | NPB (20 nm) | 8.9 | 5.8 | x=0.16 |
| | | | | | y=0.24 |
| Beispiel 61 | **B37 (20 nm)** | NPB (20 nm) | 7.9 | 6.3 | x=0.16 |
| | | | | | y=0.25 |
| Beispiel 62 | **B37 (100 nm)** | NPB (20 nm) | 8.0 | 6.5 | x=0.16 |
| | | | | | y=0.24 |

### Beispiel 63: Herstellung von OLEDs mit Indenofluoren-Diaminen als Lochinjektionsmaterial bzw. Lochtransportmaterial in phosphoreszierenden OLEDs

Die Herstellung von OLEDs erfolgt nach einem allgemeinen Verfahren gemäß WO 04/093207, das im Einzelfall auf die jeweiligen Gegebenheiten (z. B. Schichtdickenvariation, um optimale Effizienz bzw. Farbe zu erreichen) angepasst wird.

In den folgenden Beispielen 64-68 werden die Ergebnisse verschiedener OLEDs vorgestellt. Der grundlegende Aufbau und die verwendeten Materialien (außer der Lochtransportschicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM1) | B2 (Verbindung nach Beispiel 2) |
| oder: | als Vergleichsbeispiel 4,4',4"-Tris(N-1-naphthyl-N-phenylamino)-triphenylamin (abgekürzt als NaphDATA bezogen von SynTec) (Vergleichsstandard) |
| Lochtransportschicht (HTM2) | 20 nm (aufgedampft; S-TAD hergestellt nach WO99/12888; 2,2',7,7'-Tetrakis-(diphenylamino)spirobifluoren) |
| oder: | 20 nm NPB (N-Naphthyl-N-phenyl-4,4'-diaminobiphenyl) |
| Emissionschicht (EML) | 40 nm **Keton-1** (Bis(9,9'-spirobifluoren-2-yl)keton (aufgedampft, synthetisiert nach WO 04/093207) dotiert mit 15 % Triplett-Emitter **E1** (synthetisiert nach WO 04/085449) |
| AlQ₃ | 20 nm (aufgedampft; AlQ₃ bezogen von SynTec; Tris(chinolinolato)aluminium(III)), |
| Kathode | 1 nm LiF, darauf 150 nm Al. |

Die OLED können ebenso ohne PEDOT als Lochinjektionsschicht hergestellt werden. In diesen Fällen ist dann das erfindungsgemäße Indenofluoren-Diamin-Derivat die Lochinjektionsverbindung. Diese OLEDs zeigen vergleichbar gute Eigenschaften.

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 2 sind die Ergebnisse einiger OLEDs (Beispiele 64 bis 68), bei denen die Schichtdicke der Lochtransportschicht (HTM1) variiert wird, zusammengefasst. Als Vergleichsmaterial wird in den Vergleichsbeispielen NaphDATA verwendet.

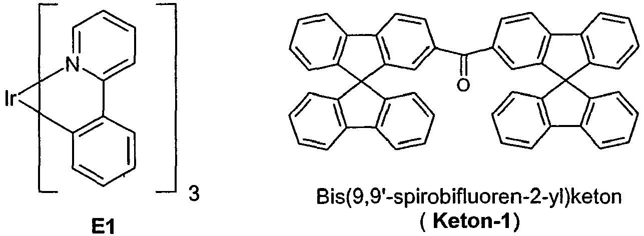

Wie man den erfindungsgemäßen Beispielen 65 bis 68 in der Tabelle 2 entnehmen kann, zeigen OLEDs enthaltend das erfindungsgemäße Lochtransportmaterial (HTM1) eine deutlich niedrigere Betriebsspannung und eine höhere Effizienz als mit NaphDATA gemäß dem Stand der Technik als Lochtransportmaterial. Weiterhin ist die Betriebsspannung unabhängig von der Schichtdicke der Lochtransportschicht. Das erfindungsgemäße Lochtransportmaterial kann hier also als Dickenausgleichsschicht dienen, ohne dass dadurch die elektooptischen Eigenschaften des Devices nachteilig beeinflusst werden.

**Tabelle 2**

| **Beispiel** | **HTL 1 or HIL** | **HTL 2** | **Max. Effizienz (cd/A)** | **Spannung (V) bei 1000cd/m**² | **CIE** |
|---|---|---|---|---|---|
| Beispiel 64 (Vergleich) | NaphDATA (20 nm) | S-TAD (20 nm) | 33 | 4.5 | x=0.38 |
| | | | | | y=0.58 |
| Beispiel 65 | **B2 (20 nm)** | S-TAD (20 nm) | 42 | 3.8 | x=0.38 |
| | | | | | y=0.58 |
| Beispiel 66 | **B2 (150 nm)** | S-TAD (20 nm) | 40 | 3.9 | x=0.36 |
| | | | | | y=0.60 |
| Beispiel 67 | **B2 (20 nm)** | NPB (20 nm) | 37 | 4.2 | x=0.38 |
| | | | | | y=0.58 |
| Beispiel 68 | **B₂ (150 nm)** | NPB (20 nm) | 35 | 4.3 | x=0.36 |
| | | | | | y=0.60 |

### Beispiel 69: Herstellung von OLEDs mit Indenofluorenketonen oder Indenofluorenphosphinoxiden als Elektronentransportmaterial in phosphoreszierenden OLEDs

Die Herstellung von OLEDs erfolgt nach einem allgemeinen Verfahren gemäß WO 04/093207, das im Einzelfall auf die jeweiligen Gegebenheiten (z. B. Schichtdickenvariation, um optimale Effizienz bzw. Farbe zu erreichen) angepasst wird.

In den folgenden Beispielen 70-73 werden die Ergebnisse verschiedener OLEDs vorgestellt. Der grundlegende Aufbau und die verwendeten Materialien (außer der Elekronentransportschicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. |
| | Starck, Goslar, Deutschland; Poly(3,4-ethylendioxy-2,5-thiophen)) |
| Lochtransportschicht (HTM1) | 20nm 4,4',4"-Tris(N-1-naphthyl-N-phenyl-amino)-triphenylamin (abgekürzt als NaphDATA bezogen von SynTec) |
| Lochtransportschicht (HTM2) | 20 nm S-TAD (aufgedampft; S-TAD hergestellt nach WO99/12888; 2,2',7,7'-Tetrakis(diphenylamino)-spirobifluoren) |
| Emissionschicht (EML) | 40 nm **Keton-1** (Bis(9,9'-spirobifluoren-2-yl)keton (aufgedampft, synthetisiert nach WO 04/093207), dotiert mit 15 % Triplett-Emitter E1 (synthetisiert nach WO 04/085449) |
| Elekronentransportschicht | 20nm **B19** (Verbindung nach Beispiel 19) |
| oder | 20nm **B26** (Verbindung nach Beispiel 26) |
| oder | 20nm **B31** (Verbindung nach Beispiel 31) |
| oder | 20 nm AlQ₃ (AlQ₃ bezogen von SynTec; Tris(chinolinolato)aluminium(III), Vergleich) |
| Kathode | 1 nm LiF, darauf 150 nm Al. |

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 3 sind die Ergebnisse einiger OLEDs (Beispiele 70 bis 73), bei denen die Elektronenetransportschicht (ETL) variiert wird, zusammengefasst. Als Vergleichsmaterial wird in den Vergleichsbeispielen Alq verwendet.

Der Emitter **E1** und das Matrixmaterial **Keton-1** sind in Beispiel 63 abgebildet.

Wie man den erfindungsgemäßen Beispielen 71 bis 73 in der Tabelle 3 entnehmen kann, zeigen OLEDs enthaltend das erfindungsgemäße Elektronentransportmaterial eine niedrigere Betriebsspannung und eine höhere Effizienz als mit Alq gemäß dem Stand der Technik.

**Tabelle 3**

| **Beispiel** | **ETL** | **Max. Effizienz (cd/A)** | **Spannung (V) bei 1000cd/m²** | **CIE** |
|---|---|---|---|---|
| Beispiel 70 (Vergleich) | Alq (20 nm) | 33 | 4.5 | x=0.38; y=0.58 |
| Beispiel 71 | **B19 (20 nm)** | 35 | 4.2 | x=0.38; y=0.58 |
| Beispiel 72 | **B26 (20 nm)** | 38 | 3.9 | x=0.38; y=0.58 |
| Beispiel 73 | **B31 (20 nm)** | 37 | 4.1 | x=0.38; y=0.58 |

### Beispiel 74: Herstellung von rot phosphoreszierenden OLEDs mit Indenofluorenketonen oder Indenofluorenphosphinoxiden als Triplett-Matrixmaterial

Die Herstellung von OLEDs erfolgt nach einem allgemeinen Verfahren gemäß WO 04/093207, das im Einzelfall auf die jeweiligen Gegebenheiten (z. B. Schichtdickenvariation, um optimale Effizienz bzw. Farbe zu erreichen) angepasst wird.

In den folgenden Beispielen 75-78 werden die Ergebnisse verschiedener OLEDs vorgestellt. Der grundlegende Aufbau und die verwendeten Materialien (außer der Elekronentransportschicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM1) | 20nm 4,4',4"-Tris(N-1-naphthyl-N-phenyl-amino)-triphenylamin (abgekürzt als NaphDATA bezogen von SynTec) |
| Lochtransportschicht (HTM2) | 20 nm S-TAD (aufgedampft; S-TAD hergestellt nach WO99/12888; 2,2',7,7'-Tetrakis(diphenylamino)-spirobifluoren) |
| Emissionschicht (EML) | 20nm B19 (Verbindung nach Beispiel 26) |
| oder | 20nm B26 (Verbindung nach Beispiel 31) |
| oder | 20nm B31 (Verbindung nach Beispiel 31) |
| oder | **Keton-1** (Bis(9,9'-spirobifluoren-2-yl)keton (aufgedampft, synthetisiert nach WO 04/093207) (Vergleichsstandard), jeweils dotiert mit 10 % Triplett-Emitter **E2** (synthetisiert nach WO 05/033244) |
| Elekronentransportschicht | 20 nm AlQ₃ (aufgedampft; AlQ₃ bezogen von SynTec; Tris(chinolinolato)aluminium(III)), |
| Kathode | 1 nm LiF, darauf 150 nm Al. |

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 4 sind die Ergebnisse einiger OLEDs (Beispiele 75 bis 78), bei denen das Triplett-Matrixmaterial der Emissionschicht (EML) variiert wird, zusammengefasst. Als Vergleichsmaterial wird in den Vergleichsbeispielen **Keton-1** verwendet.

Der Emitter **E1** und das Triplett-Matrixmaterial **Keton-1** sind im Folgenden der Übersichtlichkeit halber abgebildet:

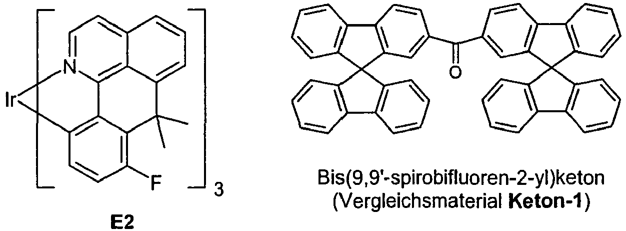

Wie man den erfindungsgemäßen Beispielen 76 bis 78 in der Tabelle 4 entnehmen kann, zeigen OLEDs enthaltend das erfindungsgemäße Triplett-Matrixmaterial eine niedrigere Betriebsspannung und eine höhere Effizienz als mit **Keton-1** gemäß dem Stand der Technik.

**Tabelle 4**

| **Beispiel** | **EML Host: Emitter** | **Max. Effizienz (cd/A)** | **Spannung (V) bei 1000cd/m²** | **CIE** |
|---|---|---|---|---|
| Beispiel 75 (Vergleich) | E2 : Keton 1 (40 nm) | 13.3 | 5.5 | x=0.65; y=0.35 |
| Beispiel 76 | **E2 : B19 (40 nm)** | 15.4 | 5.2 | x=0.65; y=0.35 |
| Beispiel 77 | **E2 : B26 (40 nm)** | 14.8 | 5.4 | x=0.65; y=0.35 |
| Beispiel 78 | **E2 : B31 (40 nm)** | 14.5 | 5.1 | x=0.65; y=0.35 |

### Beispiel 79: Herstellung von OLEDs mit Indenofluoren-Diaminen als Emitter

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 80-86 werden die Ergebnisse verschiedener OLEDs vorgestellt. Der grundlegende Aufbau und die verwendeten Materialien (außer der emittierenden Schicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM1) | 20 nm **B2** (Verbindung nach Beispiel 2); |
| Lochtransportschicht (HTM2) | 20 nm NPB (N-Naphthyl-N-phenyl-4,4'-diaminobiphenyl) |
| Emissionschicht (EML) | 30 nm, Schicht aus **H1**, **H2** oder **H3** als Hostmaterial dotiert mit x % (s. Tabelle) **B2** (Verbindung nach Beispiel 2) oder **B17** (Verbindung nach Beispiel 17) als Dotand |
| Elektronenleiter (ETL) | 20 nm (aufgedampft; AlQ₃ bezogen von SynTec; Tris(chinolinolato)aluminium(III)) |
| Kathode | 1 nm LiF, darauf 150 nm Al. |

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 5 sind die Ergebnisse einiger OLEDs (Beispiele 80 bis 86), bei denen **B2** (Verbindung nach Beispiel 2) oder **B17** (Verbindung nach Beispiel 17) als tiefblauer Emitter verwendet und dessen Dotierungsgrad variiert wird, zusammengefasst.

Die Hostmaterialien **H1, H2** und **H3** sind im Folgenden dargestellt:

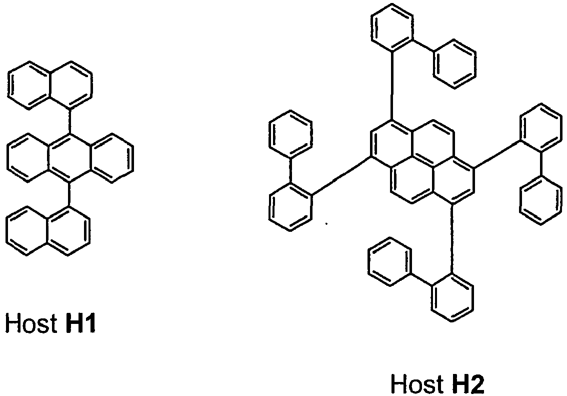

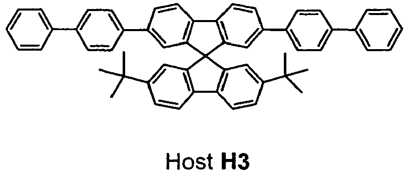

Wie man den Beispielen 80 bis 86 in der Tabelle 5 entnehmen kann, zeigen OLEDs enthaltend die erfindungsgemäßen Dotanden **B2** (Verbindung nach Beispiel 2) und **B17** (Verbindung nach Beispiel 17) effiziente tiefblaue Emission. Dagegen werden mit kommerziellen OLEDs lediglich Farbkoordinaten von (0.15; 0.15) erreicht werden. Die interne Quanteneffizient liegt nahe bei 100 %.

**Tabelle 5**

| **Beispiel** | **EML** | **Max Effizienz (cd/A)** | **Spannung (V) bei 1000cd/m²** | **CIE** |
|---|---|---|---|---|
| Beispiel 80 | **H2** | 2.5 | 6.6 | x=0.16 |
| | 2 % **B2** | | | y=0.09 |
| Beispiel 81 | **H2** | 2.8 | 6.4 | x=0.16 |
| | 5 % **B2** | | | y=0.10 |
| Beispiel 82 | **H2** | 2.7 | 6.3 | x=0.16 |
| | 10 % **B2** | | | y=0.13 |
| Beispiel 83 | **H3** | 2.3 | 6.8 | x=0.16 |
| | 5 % **B2** | | | y=0.04 |
| Beispiel 84 | **H3** | 2.2 | 6.5 | x=0.16 |
| | 10 % **B2** | | | y=0.05 |
| Beispiel 85 | **H3** | 2.0 | 6.3 | x=0.16 |
| | 15 % **B2** | | | y=0.05 |
| Beispiel 86 | **H1** | 2.4 | 6.2 | x=0.16 |
| | 10 % **B17** | | | y=0.08 |

### Beispiel 87: Bis(N-(4-tert-butylphenyl)-N-(4-bromphenyl)amino)-6,6,12,12-tetraoctyl-6,12,dihydroindeno[1,2b]fluoren

### a) Bis(N-(4-tert-butylphenyl)-N-phenylamino)-6,6,12,12-tetraoctyl-6,12,dihydroindeno[1,2b]fluoren

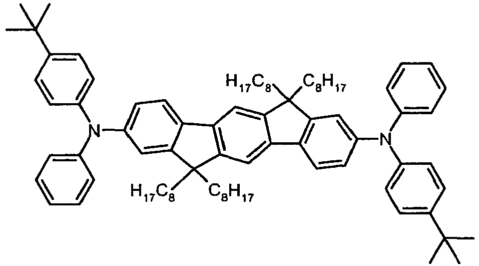

Eine Lösung aus 17.2 g (20.0 mmol) 6,6,12,12-Tetraoctyl-6,12-dihydro-[1,2b]indenofluorendibromid und 10.0 g (44.4 mmol) 4-tert-Butylphenylphenylamin in 130 ml trockenem Toluol wird mit Argon gesättigt. Dann werden 81.0 mg Tri-tert-butylphosphin, 45 mg Palladiumacetat und 5.97 g Natrium-tert-butoxid zugegeben. Die Reaktionsmischung wird 12.5 h unter Rückfluss erhitzt. Nach Abkühlen auf RT wird mit 2M HCl (2 x 100 ml) extrahiert. Die organische Phase wird abgetrennt, über Celite filtriert und einrotiert. Das Rohprodukt wird aus EtOH/Toluol umkristallisiert. Es werden 18.6 g (81%) gelbe Kristalle erhalten.

### b) Bis(N-(4-tert-butylphenyl)-N-(4-bromphenyl)amino)-6,6,12,12-tetraoctyl-6,12,dihydroindeno[1,2b]fluoren

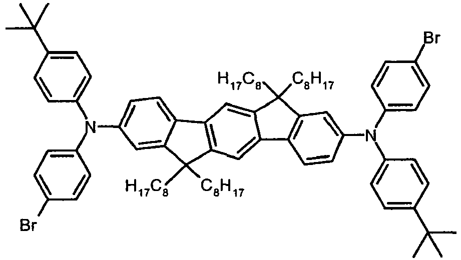

5.0 g (3.8 mmol) Bis(N-(4-tert-butylphenyl)-N-phenylamino)-6,6,12,12-tetraoctyl-6,12,dihydroindeno[1,2b]fluoren werden in 22.4 ml trockenem THF gelöst und bei 0 °C tropfenweise mit einer Lösung aus 1.5 g NBS in 22.4 ml THF versetzt. Man lässt die Reaktionsmischung auf RT kommen und entfernt das Lösemittel. Der Feststoff wird mit Ethanol ausgekocht und abgesaugt. Das Rohprodukt wird nach dem Trocknen im Vakuum aus Acetonitril/Toluol umkristallisiert. Es werden 2.16 g (43%) hellgelbe Kristalle isoliert.

Bis(N-(4-tert-butylphenyl)-N-(4-bromphenyl)amino)-6,6,12,12-tetraoctyl-6,12,dihydroindeno[1,2b]fluoren lässt sich als Monomer zur Polymerisation, beispielsweise zur Polymerisation gemäß Suzuki oder gemäß Yamamoto, einsetzen. Diese Verbindung ist besonders gut geeignet für den Einbau in konjugierte oder teilkonjugierte Polymere und eignet sich insbesondere als lochleitende Verbindung in diesen Polymeren.

### Beispiel 88: 2,10-Bis(diphenylamino)-12,15-dihydro-6,6,12,12,15,15-hexamethyl-6H-diindeno[1,2-b:2',1'-h]fluoren

### a) 12,15-Dihydro-6,6,12,12,15,15-hexamethyl-6H-diindeno-[1,2-b:2',1'-h]fluoren

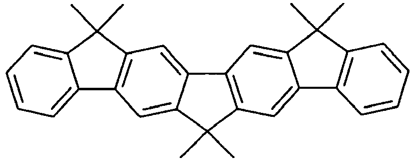

Die Darstellung erfolgt analog zur Darstellung von 9,9-Dimethylfluoren aus 12,15-Dihydro-6H-diindeno[1,2-b:2',1'-h]fluoren (Stauner et al., Helv. Chim. Acta 1970, 53(6), 1311), Dimethylsulfat und Natronlauge nach JP 08113542. Ausbeute 61.0 % d. Th.; Reinheit 97 % n. ¹H-NMR.

### b) 2,10-Dibrom-12,15-dihydro-6,6,12,12,15,15-hexamethyl-6H-diindeno [1,2-b:2',1'-h]fluoren

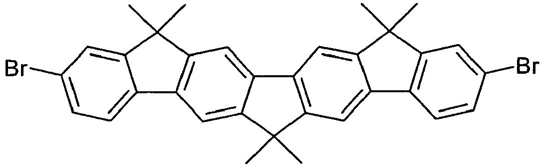

Darstellung analog Beispiel 1b. Anstelle von 122.0 g (393 mmol) 6,6,12,12-Tetramethyl-6,12-dihydro-indeno[1,2b]fluoren werden 167.7 g (393 mmol) 12,15-Dihydro-6,6,12,12,15,15-hexamethyl-6H-diindeno-[1,2-b:2',1'-h]fluoren eingesetzt. Ausbeute: 198.5 g (339 mmol), 86.4 % d. Th.; Reinheit: 98 % n. ¹H-NMR.

### c) 2,10-Bis(diphenylamino)-12,15-dihydro-6,6,12,12,15,15-hexamethyl-6H-diindeno[1,2-b:2',1-h]fluoren

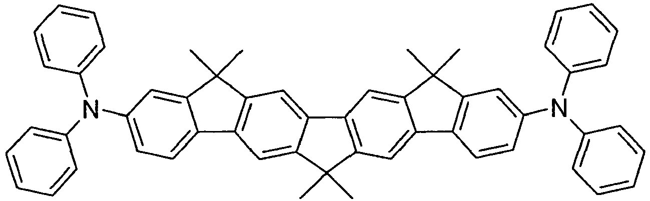

Darstellung analog Beispiel 1c. Anstelle von 46.8 g (100 mmol) 2,8-Dibrom-6,6,12,12-tetramethyl-6,12-dihydroindeno[1,2b]fluoren werden 58.4 g (100 mmol) 2,10-Dibrom-12,15-dihydro-6,6,12,12,15,15-hexamethyl-6H-diindeno[1,2-b:2',1'-h]fluoren verwendet. Sublimation p = 1 × 10⁻⁵ mbar, T = 390 °C. Ausbeute: 55.0 g (72 mmol), 72.3 % d. Th.; Reinheit: 99.9 % n. HPLC.

### Beispiel 89: 2,8-Bis(bis(4-diphenylaminophenyl)amino)-6,6,12,12-tetramethyl-6,12-dihydro-indeno[1,2b]fluoren

### a) 2,8-Bis(bis(4-bromphenyl)amino)-6,6,12,12-tetramethyl-6,12-dihydro-indeno[1,2b]fluoren

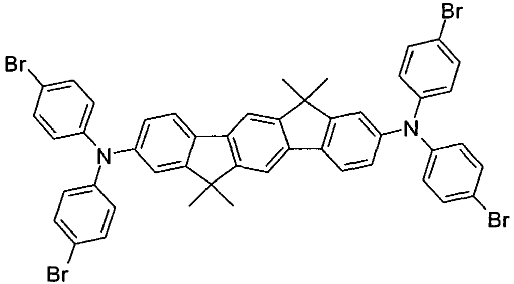

Eine Lösung von 64.5 g (100 mmol) 2,8-Bis(diphenylamino)-6,6,12,12-tetramethyl-6,12-dihydroindeno[1,2b]fluoren in 1500 ml Dichlormethan wird unter gutem Rühren portionsweise mit 74.8 g (420 mmol) N-Bromsuccinimid versetzt und 16 h bei Raumtemperatur gerührt. Die Reaktionsmischung wird im Vakuum auf 200 ml Volumen eingeengt, mit 1000 ml Ethanol versetzt, der Niederschlag wird abgesaugt, mit 1000 ml heißem Ethanol ausgerührt, abgesaugt, dreimal mit je 300 ml Ethanol gewaschen und im Vakuum getrocknet. Ausbeute: 82.1 g (85 mmol), 85.5 % d. Th.; Reinheit: 97 % n. ¹H-NMR.

### b) 2,8-Bis(bis(4-diphenylaminophenyl)amino)-6,6,12,12-tetramethyl-6,12-dihydro-indeno[1,2b]fluoren

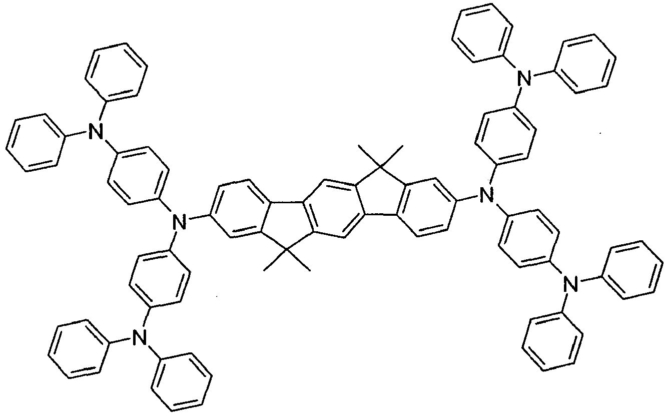

Durchführung analog zu Beispiel 1c. Anstelle von 46.8 g (100 mmol) 2,8-Dibrom-6,6,12,12-tetramethyl-6,12-dihydroindeno[1,2b]fluoren werden 48.0 g (50 mmol) 2,8-Bis(bis(4-bromphenyl)amino)-6,6,12,12-tetramethyl-6,12-dihydro-indeno[1,2b]fluoren eingesetzt. Umkristallisation aus Dioxan; Sublimation p = 1 × 10⁻⁵ mbar, T = 380 °C. Ausbeute: 48.8 g (37 mmol), 74.3 % d. Th.; Reinheit: 99.8 % n. HPLC.

### Beispiel 90: 2,8-Bis((4-methylphenyl)(4-diphenylaminophenyl)amino)-6,6,12,12-tetramethyl-6,12-dihydro-indeno[1,2b]fluoren

### a) 2,8-Bis((4-bromphenyl)(4-methylphenyl)amino)-6,6,12,12-tetramethyl-6,12-dihydro-indeno[1,2b]fluoren

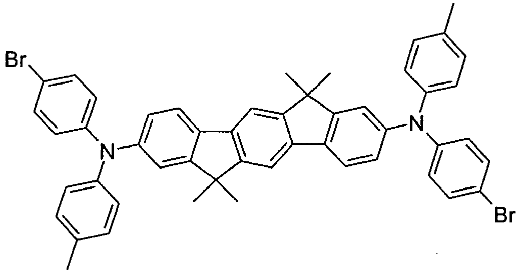

Eine Lösung von 134.6 g (200 mmol) 2,8-Bis((phenyl)(4-methylphenyl)-amino)-6,6,12,12-tetramethyl-6,12-dihydro-indeno[1,2b]fluoren (Darstellung analog zu Beispiel 1c) in 1500 ml Dichlormethan wird unter gutem Rühren portionsweise mit 74.8 g (420 mmol) N-Bromsuccinimid versetzt und 16 h bei Raumtemperatur gerührt. Die Reaktionsmischung wird im Vakuum auf 200 ml Volumen eingeengt, mit 1000 ml Ethanol versetzt, der Niederschlag wird abgesaugt, mit 1000 ml heißem Ethanol ausgerührt, abgesaugt, dreimal mit je 300 ml Ethanol gewaschen und im Vakuum getrocknet. Ausbeute: 139.0 g (167 mmol), 83.6 % d. Th.; Reinheit: 98 % n. ¹H-NMR.

### b) 2,8-Bis((4-methylphenyl)(4-diphenylaminophenyl)amino)-6,6,12,12-tetramethyl-6,12-dihydro-indeno[1,2b]fluoren

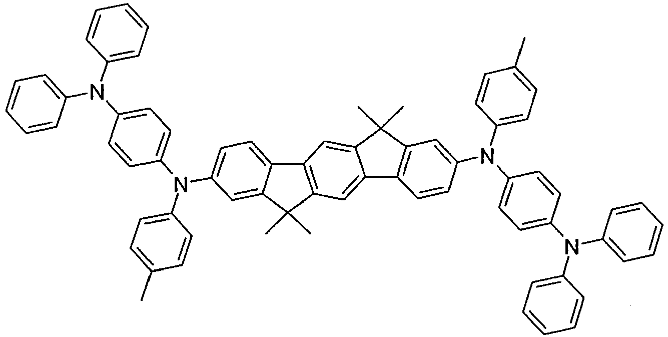

Durchführung analog zu Beispiel 1c. Anstelle von 46.8 g (100 mmol) 2,8-Dibrom-6,6,12,12-tetramethyl-6,12-dihydroindeno[1,2b]fluoren werden 83.1 g (100 mmol) 2,8-Bis((4-bromphenyl)(4-methylphenyl)amino)-6,6,12,12-tetramethyl-6,12-dihydroindeno[1,2b]fluoren eingesetzt. Umkristallisation aus NMP; Sublimation p = 1 x 10⁻⁵ mbar, T = 370 °C. Ausbeute: 83.6 g (83 mmol), 82.9 % d. Th.; Reinheit: 99.7 % n. HPLC.

### Beispiel 91: Herstellung von OLEDs mit Indenofluoren-Tetraminen bzw. -Hexaminen als Lochinjektionsmaterial bzw. Lochtransportmaterial in fluoreszierenden OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 92-94 werden die Ergebnisse verschiedener OLEDs vorgestellt. Der grundlegende Aufbau und die verwendeten Materialien (außer der Lochtransportschicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM1) | **B89** (Verbindung nach Beispiel 89) |
| oder | **B90** (Verbindung nach Beispiel 90) |
| oder | als Vergleichsbeispiel 4,4',4"-Tris(N-1-naphthyl-N-phenylamino)-triphenylamin (abgekürzt als NaphDATA bezogen von SynTec) |
| Lochtransportschicht (HTM2) | 20 nm NPB (N-Naphthyl-N-phenyl-4,4'-diaminobiphenyl) |
| Emissionschicht (EML) | 30 nm, dotierte Schicht aus 9,10-Bis(1-naphthylanthracen) als Hostmaterial (abgekürzt **H1**), dotiert mit 5 % Tris[4-(2,2-diphenylvinyl)-phenyl]amin als Dotand (abgekürzt **D1,** aufgedampft, synthetisiert nach WO 06/000388) |
| Elektronenleiter (ETL) | 20 nm AlQ₃ (bezogen von SynTec, Tris(chinolinato)aluminium(III)) |
| Kathode | 1 nm LiF, darauf 150 nm Al. |

Die OLED können ebenso ohne PEDOT als Lochinjektionsschicht hergestellt werden. In diesen Fällen ist dann das erfindungsgemäße Indenofluoren-Tetramin bzw. Hexamin-Derivat die Lochinjektionsverbindung. Diese OLEDs zeigen vergleichbar gute Eigenschaften.

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 6 sind die Ergebnisse einiger OLEDs (Beispiele 92 bis 94), bei denen die Schichtdicke der Lochtransportschicht (HTM1) variiert wird, zusammengefasst. Als Vergleichsmaterial wird in den Vergleichsbeispielen NaphDATA verwendet.

Das Hostmaterial **H1** ist 9,10-Bis(1-naphthyl)anthracen, als Dotand wird **D1** eingesetzt. Beide sind im Folgenden dargestellt:

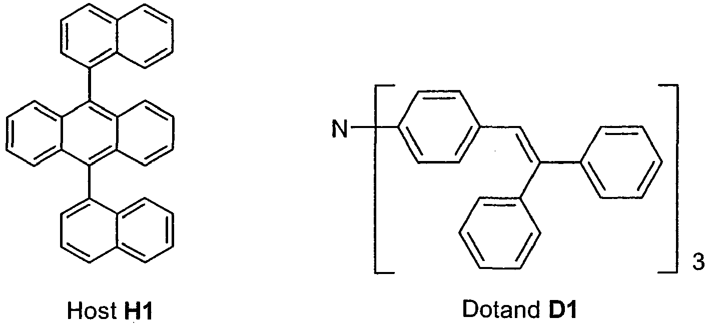

Wie man den erfindungsgemäßen Beispielen 92 und 93 in der Tabelle 6 entnehmen kann, zeigen OLEDs enthaltend das erfindungsgemäße Lochtransportmaterial (HTM1) eine deutlich höhere Effizienz als mit NaphDATA gemäß dem Stand der Technik als Lochtransportmaterial. Weiterhin ist die Betriebsspannung unabhängig von der Schichtdicke der Lochtransportschicht. Diese Eigenschaft ist für den Aufbau vollfarbiger Displays von großem Vorteil, da man durch Variation der Schichtdicke der Lochtransportschicht die Dicke der Pixel der Grundfarben Blau, Grün und Rot gleich gestalten kann. Das erfindungsgemäße Lochtransportmaterial kann hier also als Dickenausgleichsschicht dienen, ohne dass dadurch die elektooptischen Eigenschaften des Devices nachteilig beeinflusst werden. Wie den Vergleichsbeispielen zu entnehmen ist, ist dies für Lochtransportmaterial (NaphDATA) gemäß dem Stand der Technik nicht der Fall: Hier wird bei größerer Schichtdicke der Lochtransportschicht eine deutlich höhere Betriebsspannung benötigt.

**Tabelle 6**

| **Beispiel** | **HTL 1 or HIL** | **HTL 2** | **Max. Effizienz (cd/A)** | **Spannung (V) bei 1000cd/m²** | **CIE** |
|---|---|---|---|---|---|
| Beispiel 92 | **B89** (20 nm) | NPB (20 nm) | 8.9 | 5.4 | x=0.16 |
| | | | | | y=0.25 |
| Beispiel 93 | **B90** (20 nm) | NPB (20 nm) | 9.0 | 5.5 | x=0.16 |
| | | | | | y=0.25 |
| Beispiel 94 (Vergleich) | NaphDATA (20 nm) | NPB (20 nm) | 7.5 | 6.1 | x=0.16 |
| | | | | | y=0.25 |

## Patentansprüche

1. Verbindungen gemäß Formel (1), 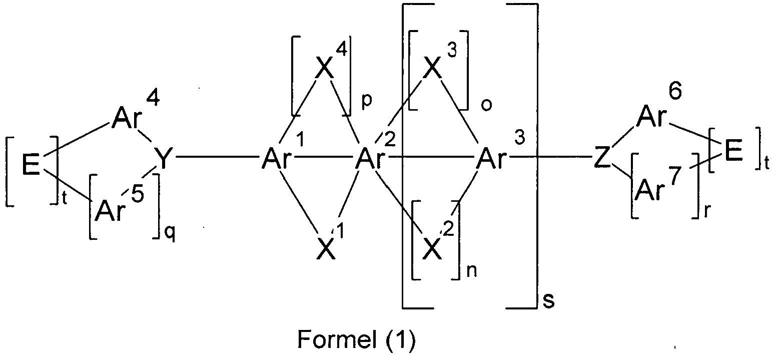 wobei für die verwendeten Symbole und Indizes gilt:
Y, Z ist gleich oder verschieden N, P, P=O, PF₂, P=S, As, As=O, As=S, Sb, Sb=O, Sb=S, C=O, O, S, Se, Te, S=O, SO₂, Se=O, SeO₂, Te=O oder TeO₂;
Ar¹, Ar², Ar³ ist Benzol, das mit einem oder mehreren Resten R¹ substituiert sein kann;
Ar⁴, Ar⁵, Ar⁶, Ar⁷ ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
E ist bei jedem Auftreten gleich oder verschieden eine Einfachbindung, N(R¹), O, S, C(R¹)₂, Si(R¹)₂ oder B(R¹);
R¹ ist bei jedem Auftreten gleich oder verschieden H, F, Cl, Br, I, CN, NO₂, Si(R²)₃, B(OR²)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die mit einem oder mehreren Resten R² substituiert sein kann, wobei eine oder mehrere nicht benachbarte CH₂-Gruppen durch -C=C-, Si(R²)₂, Ge(R²)₂, Sn(R²)₂, C=O, C=S, C=Se, C=NR², -O-, -S-, -COO- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das durch einen oder mehrere nicht-aromatische Reste R¹ substituiert sein kann, oder eine Aryloxy- oder Heteroaryloxygruppe mit 5 bis 40 aromatischen Ringatomen, die durch einen oder mehrere nicht-aromatische Reste R¹ substituiert sein kann, oder eine Kombination dieser Systeme; dabei können zwei oder mehrere Substituenten R¹ auch miteinander ein mono- oder polycyclisches Ringsystem bilden;
R² ist bei jedem Auftreten gleich oder verschieden H oder ein aliphatischer oder aromatischer Kohlenwasserstoffrest mit 1 bis 20 C-Atomen;
X¹, X⁴ ist bei jedem Auftreten gleich oder verschieden eine Brücke, die mit Ar¹ und Ar² ein cyclisches System aufspannt, ausgewählt aus C(R¹)₂, Si(R¹)₂, C=O, C=NR¹, C=C(R¹)₂, O, S, S=O, SO₂, N(R¹), P(R¹), P(=O)R¹, P(=S)R¹, B(R¹) oder einer Kombination aus zwei, drei oder vier dieser Gruppen;
X², X³ ist bei jedem Auftreten gleich oder verschieden eine Brücke, die mit Ar² und Ar³ ein cyclisches Ringsystem aufspannt, ausgewählt aus B(R¹), C(R¹)₂, Si(R¹)₂, C=O, C=NR¹, C=C(R¹)₂, O, S, S=O, SO₂, N(R¹), P(R¹), P(=O)R¹, P(=S)R¹ oder einer Kombination aus zwei, drei oder vier dieser Gruppen;
p, n, o p ist null und einer der beiden Indizes n oder o ist eins, während der andere der beiden Indizes n oder o null ist, dabei bedeutet n = 0 bzw. o = 0 bzw. p = 0, dass sich an Stelle der Brücke zwei H bzw. Reste R¹ befinden;
q, r ist bei jedem Auftreten 1, wenn das entsprechende Zentralatom der Gruppe Y bzw. Z ein Element aus der 5. Hauptgruppe ist, und ist bei jedem Auftreten gleich 0, wenn das entsprechende Zentralatom der Gruppe Y bzw. Z ein Element aus der 4. oder der 6. Hauptgruppe ist;
s ist 1, 2 oder 3;
t ist bei jedem Auftreten gleich oder verschieden 0 oder 1, wobei t = 0 bedeutet, dass statt der Gruppe E Reste R¹ gebunden sind; dabei gilt weiterhin, dass t = 0 ist, wenn q = 0 ist.

2. Verbindungen gemäß Anspruch 1, **dadurch gekennzeichnet, dass** die Symbole Y, Z gleich oder verschieden für Stickstoff, C=O, Phosphor oder P=O steht.

3. Verbindungen der Formel (1a) gemäß Anspruch 2, 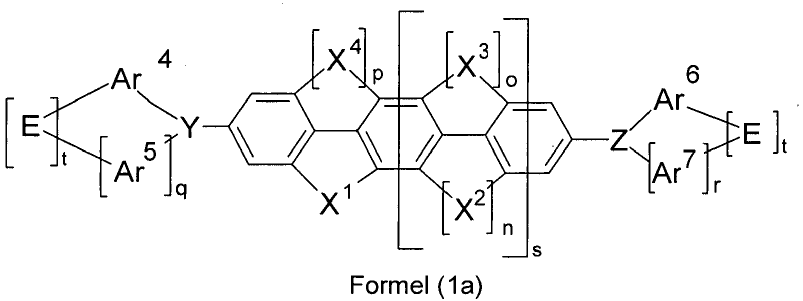 wobei die Symbole und Indizes dieselben Bedeutungen haben, wie in Anspruch 1 beschrieben.

4. Verbindungen gemäß einem oder mehreren der Ansprüche 1 bis 3, **dadurch gekennzeichnet, dass** die Symbole Ar⁴, Ar⁵, Ar⁶, Ar⁷ gleich oder verschieden bei jedem Auftreten für ein aromatisches oder heteroaromatisches Ringsystem mit 5 bis 16 aromatischen Ringatomen, für ein Triarylamin oder für Spirobifluoren stehen, das jeweils mit einem oder mehreren Resten R¹ substituiert sein kann.

5. Verbindungen gemäß einem oder mehreren der Ansprüche 1 bis 4, **dadurch gekennzeichnet, dass** das Symbol R¹ gleich oder verschieden bei jedem Auftreten für H, F, CN, eine geradkettige Alkylgruppe mit 1 bis 5 C-Atomen oder eine verzweigte Alkylgruppe mit 3 bis 5 C-Atomen, wobei jeweils eine oder mehrere nicht benachbarte CH₂-Gruppen durch -C≡C-, -O- oder -S- ersetzt sein können und wobei ein oder mehrere H-Atome durch F ersetzt sein können, oder eine monovalente Aryl- oder Heteroarylgruppe mit 2 bis 16 C-Atomen, die mit einem oder mehreren Resten R² substituiert sein kann, steht; dabei können auch zwei oder mehrere Reste R¹ miteinander ein Ringsystem bilden.

6. Verbindungen gemäß einem oder mehreren der Ansprüche 1 bis 5, gemäß den Strukturen der Formel (1b) und (1c), 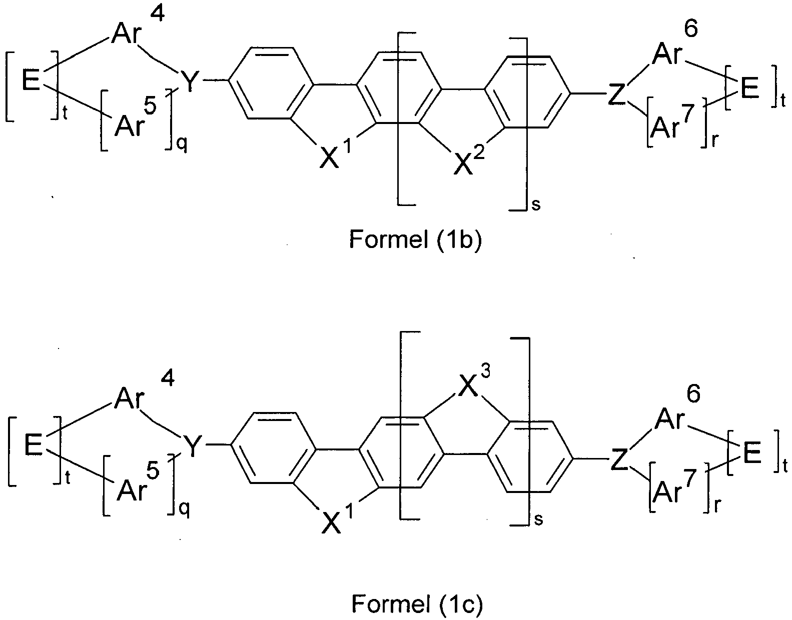 wobei die Symbole und Indizes dieselbe Bedeutung haben, wie in Anspruch 1 beschrieben.

7. Verbindungen gemäß einem oder mehreren der Ansprüche 1 bis 6, **dadurch gekennzeichnet, dass** die Symbole X¹, X², X³ und X⁴ bei jedem Auftreten gleicht oder verschieden eine Brücke darstellen, die mit Ar¹ und Ar² bzw. mit Ar² und Ar³ ein cyclisches System aufspannt, ausgewählt aus C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O und C(R¹)₂-O-C(R¹)₂.

8. Verbindungen der Formel (1d) gemäß Anspruch 7, 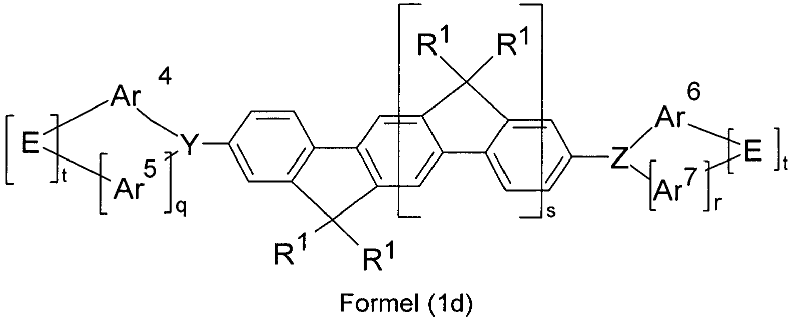 wobei die Symbole und Indizes dieselben Bedeutungen haben, wie in Anspruch 1 beischrieben.

9. Verbindungen gemäß Anspruch 8, **dadurch gekennzeichnet, dass** die Reste R¹ miteinander ein Ringsystem bilden.

10. Verbindungen gemäß einem oder mehreren der Ansprüche 1 bis 9 **dadurch gekennzeichnet, dass** Y=Z ist.

11. Verbindungen gemäß einem oder mehreren der Ansprüche 1 bis 10, ausgewählt aus den Strukturen (1) bis (104).

12. Verwendung von Verbindungen gemäß einem oder mehreren der Ansprüche 1 bis 11 in organischen elektronischen Vorrichtungen.

13. Organische elektronische Vorrichtungen, enthaltend mindestens eine Verbindung gemäß einem oder mehreren der Ansprüche 1 bis 11.

14. Organische elektronische Vorrichtung gemäß Anspruch 13, ausgewählt aus der Gruppe bestehend aus organischen Elektrolumineszenzvorrichtungen (OLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren oder organischen Laserdioden (O-Laser).

15. Organische Elektrolumineszenzvorrichtung, enthaltend Anode, Kathode und mindestens eine emittierende Schicht, **dadurch gekennzeichnet, dass** die emittierende Schicht mindestens eine Verbindung gemäß einem oder mehreren der Ansprüche 1 bis 11 enthält.

16. organische Elektrolumineszenzvorrichtung gemäß Anspruch 15, **dadurch gekennzeichnet, dass** eine Verbindung gemäß einem oder mehreren der Ansprüche 1 bis 11 als Emitter verwendet wird und das Hostmaterial der emittierenden Schicht ausgewählt ist aus den Klassen der Oligoarylene, der Oligoarylene enthaltend kondensierte aromatische Gruppen, der Oligoarylenvinylene, der polypodalen Metallkomplexe, der lochleitenden Verbindungen, der elektronenleitenden Verbindungen, der Ketone, der Phosphinoxide, der Sulfoxide oder der Atropisomere.

17. Organische Elektrolumineszenzvorrichtung gemäß Anspruch 15 oder 16 **dadurch gekennzeichnet, dass** weitere Schichten vorhanden sind, ausgewählt aus Lochinjektionsschicht, Lochtransportschicht, Lochblockierschicht, Elektronentransportschicht und/oder Elektroneninjektionsschicht.

18. Organische elektronische Vorrichtung gemäß einem oder mehreren der Ansprüche 13 bis 17, **dadurch gekennzeichnet, dass** die Verbindung gemäß einem oder mehreren der Ansprüche 1 bis 11 als Lochtransportmaterial in einer Lochtransportschicht und/oder in einer Lochinjektionsschicht eingesetzt wird und dass die Verbindung in diesen Schichten gegebenenfalls durch Elektronenakzeptor-Verbindungen dotiert sein kann.

19. Organische elektronische Vorrichtung gemäß einem oder mehreren der Ansprüche 13 bis 18, **dadurch gekennzeichnet, dass** die Verbindung gemäß einem oder mehreren der Ansprüche 1 bis 11 als Elektronentransportmaterial in einer Elektronentransportschicht und/oder als Lochblockiermaterial in einer Lochblockierschicht und/oder als Triplett-Matrixmaterial in einer emittierenden Schicht eingesetzt wird.

## Claims

1. Compounds of the formula (1) 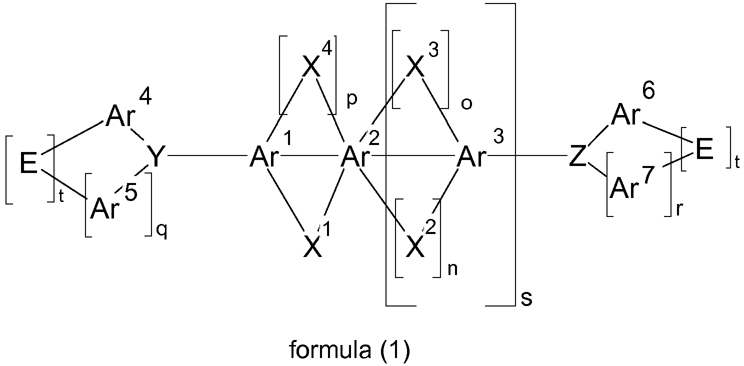 where the following applies to the symbols and indices used:
Y, Z are, identically or differently, N, P, P=O, PF₂, P=S, As, As=O, As=S, Sb, Sb=O, Sb=S, C=O, O, S, Se, Te, S=O, SO₂, Se=O, SeO₂, Te=O or TeO2;
Ar¹, Ar², Ar³ are benzene, which may be substituted by one or more radicals R¹;
Ar⁴, Ar⁵, Ar⁶, Ar⁷ are on each occurrence, identically or differently, an aromatic or heteroaromatic ring system having 5 to 40 aromatic ring atoms, which may be substituted by one or more radicals R¹;
E is on each occurrence, identically or differently, a single bond, N(R¹), O, S, C(R¹)₂, Si(R¹)₂ or B(R¹);
R¹ is on each occurrence, identically or differently, H, F, Cl, Br, I, CN, NO₂, Si(R²)₃, B(OR²)₂, a straight-chain alkyl, alkoxy or thioalkoxy group having 1 to 40 C atoms or a branched or cyclic alkyl, alkoxy or thioalkoxy group having 3 to 40 C atoms, which may be substituted by one or more radicals R², where one or more non-adjacent CH₂ groups may be replaced by -C≡C-, Si(R²)₂, Ge(R²)₂, Sn(R²)₂, C=O, C=S, C=Se, C=NR², -O-, -S-, -COO- or -CONR²- and where one or more H atoms may be replaced by F, Cl, Br, I, CN or NO₂, or an aromatic or heteroaromatic ring system having 5 to 40 aromatic ring atoms, which may be substituted by one or more non-aromatic radicals R¹, or an aryloxy or heteroaryloxy group having 5 to 40 aromatic ring atoms, which may be substituted by one or more non-aromatic radicals R¹, or a combination of these systems; two or more substituents R¹ here may also form a mono- or polycyclic ring system with one another;
R² is on each occurrence, identically or differently, H or an aliphatic or aromatic hydrocarbon radical having 1 to 20 C atoms;
X¹, X⁴ are on each occurrence, identically or differently, a bridge which, with Ar¹ and Ar², forms a cyclic system selected from C(R¹)₂, Si(R¹)₂, C=O, C=NR¹, C=C(R¹)₂, O, S, S=O, SO₂, N(R¹), P(R¹), P(=O)R¹, P(=S)R¹, B(R¹) or a combination of two, three or four of these groups;
X², X³ are on each occurrence, identically or differently, a bridge which, with Ar² and Ar³, forms a cyclic ring system selected from B(R¹), C(R¹)₂, Si(R¹)₂, C=O, C=NR¹, C=C(R¹)₂, O, S, S=O, SO₂, N(R¹), P(R¹), P(=O)R¹, P(=S)R¹ or a combination of two, three or four of these groups;
p, n, o p is zero and one of the two indices n or o is one, while the other of the two indices n or o is zero, n = 0 or o = 0 or p = 0 here means that two H or radicals R¹ are present in place of the bridge;
q, r are on each occurrence 1 if the corresponding central atom of the group Y or Z is an element from the 5th main group, and are on each occurrence equal to 0 if the corresponding central atom of the group Y or Z is an element from the 4th or 6th main group;
s is 1, 2 or 3;
t is on each occurrence, identically or differently, 0 or 1, where t = 0 means that R¹ radicals are bonded instead of the group E; furthermore, t = 0 if q = 0.

2. Compounds according to Claim 1, **characterised in that** the symbols Y, Z stand, identically or differently, for nitrogen, C=O, phosphorus or P=O.

3. Compounds of the formula (1a) according to Claim 2 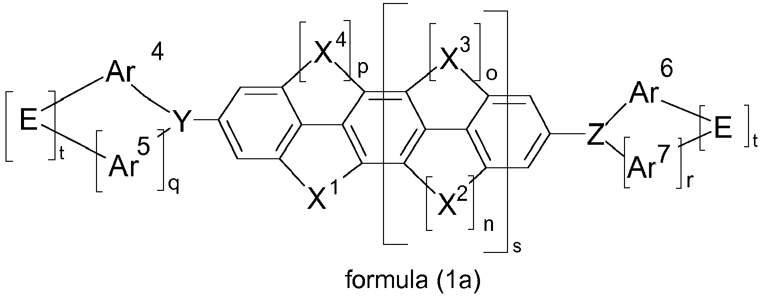 where the symbols and indices have the same meanings as described in Claim 1.

4. Compounds according to one or more of Claims 1 to 3, **characterised in that** the symbols Ar⁴, Ar⁵, Ar⁶, Ar⁷ stand, identically or differently on each occurrence, for an aromatic or heteroaromatic ring system having 5 to 16 aromatic ring atoms, for a triarylamine or for spirobifluorene, which may in each case be substituted by one or more radicals R¹.

5. Compounds according to one or more of Claims 1 to 4, **characterised in that** the symbol R¹ stands, identically or differently on each occurrence, for H, F, CN, a straight-chain alkyl group having 1 to 5 C atoms or a branched alkyl group having 3 to 5 C atoms, where in each case one or more non-adjacent CH₂ groups may be replaced by -C≡C-, -O- or -S- and where one or more H atoms may be replaced by F, or a monovalent aryl or heteroaryl group having 2 to 16 C atoms, which may be substituted by one or more radicals R²; two or more radicals R¹ here may also form a ring system with one another.

6. Compounds according to one or more of Claims 1 to 5, having the structures of the formulae (1b) and (1c), 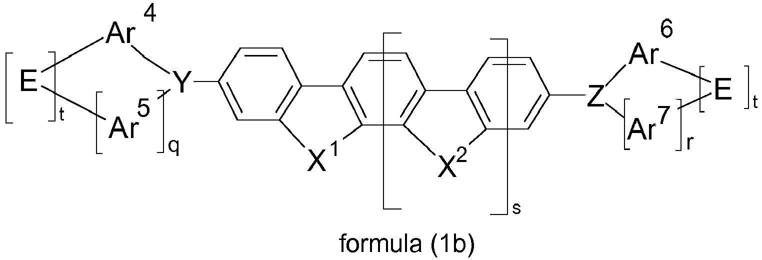 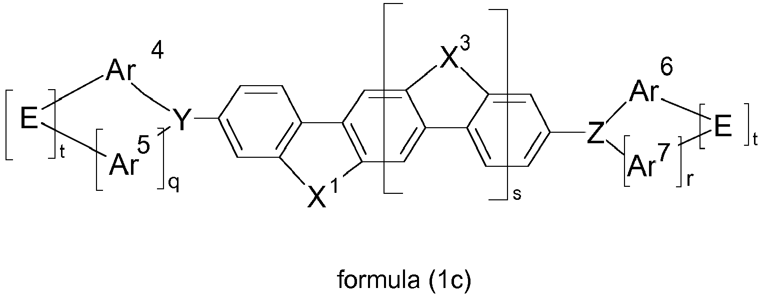 where the symbols and indices have the same meaning as described in Claim 1.

7. Compounds according to one or more of Claims 1 to 6, **characterised in that** the symbols X¹, X², X³ and X⁴ on each occurrence, identically or differently, represent a bridge which, with Ar¹ and Ar² or with Ar² and Ar³, forms a cyclic system selected from C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O and C(R¹)₂-O-C(R¹)₂.

8. Compounds of the formula (1d) according to Claim 7, 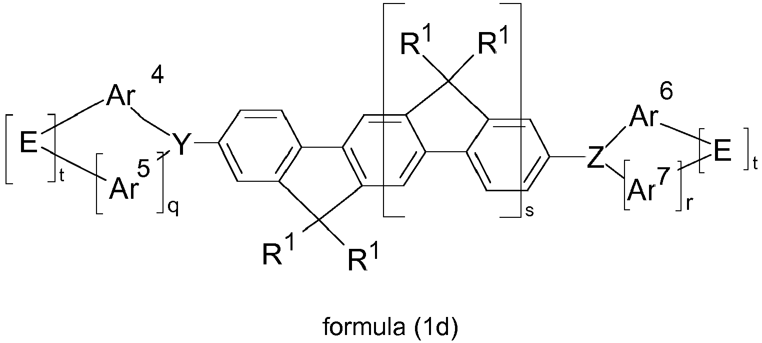 where the symbols and indices have the same meanings as described in Claim 1.

9. Compounds according to Claim 8, **characterised in that** the radicals R¹ form a ring system with one another.

10. Compounds according to one or more of Claims 1 to 9, **characterised in that** Y = Z.

11. Compounds according to one or more of Claims 1 to 10, selected from structures (1) to (104).

12. Use of compounds according to one or more of Claims 1 to 11 in organic electronic devices.

13. Organic electronic devices comprising at least one compound according to one or more of Claims 1 to 11.

14. Organic electronic device according to Claim 13, selected from the group consisting of organic electroluminescent devices (OLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic photoreceptors or organic laser diodes (O-lasers).

15. Organic electroluminescent device comprising anode, cathode and at least one emitting layer, **characterised in that** the emitting layer comprises at least one compound according to one or more of Claims 1 to 11.

16. Organic electroluminescent device according to Claim 15, **characterised in that** a compound according to one or more of Claims 1 to 11 is used as emitter, and the host material of the emitting layer is selected from the classes of the oligoarylenes, the oligoarylenes containing condensed aromatic groups, the oligoarylenevinylenes, the polypodal metal complexes, the hole-conducting compounds, the electron-conducting compounds, the ketones, the phosphine oxides, the sulfoxides or the atropisomers.

17. Organic electroluminescent device according to Claim 15 or 16, **characterised in that** further layers are present, selected from hole-injection layer, hole-transport layer, hole-blocking layer, electron-transport layer and/or electron-injection layer.

18. Organic electronic device according to one or more of Claims 13 to 17, **characterised in that** the compound according to one or more of Claims 1 to 11 is employed as hole-transport material in a hole-transport layer and/or in a hole-injection layer, and **in that** the compound in these layers may optionally be doped by electron-acceptor compounds.

19. Organic electronic device according to one or more of Claims 13 to 18, **characterised in that** the compound according to one or more of Claims 1 to 11 is employed as electron-transport material in an electron-transport layer and/or as hole-blocking material in a hole-blocking layer and/or as triplet matrix material in an emitting layer.

## Revendications

1. Composés de la formule (1) : 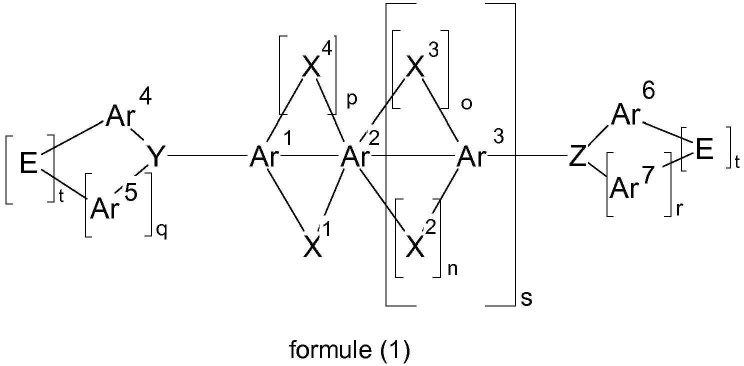 dans laquelle ce qui suit s'applique aux symboles et indices qui sont utilisés :
Y, Z sont, de manière identique ou différente, N, P, P=O, PF₂, P=S, As, As=O, As=S, Sb, Sb=O, Sb=S, C=O, O, S, Se, Te, S=O, SO₂, Se=O, SeO₂, Te=O ou TeO₂ ;
Ar¹, Ar², Ar³ sont benzène, lequel peut être substitué par un radical ou par plusieurs radicaux R¹ ;
Ar⁴, Ar⁵, Ar⁶, Ar⁷ sont pour chaque occurrence, de manière identique ou différente, un système de cycle aromatique ou hétéroaromatique qui comporte de 5 à 40 atomes de cycle aromatique, lequel peut être substitué par un radical ou par plusieurs radicaux R¹ ;
E est pour chaque occurrence, de manière identique ou différente, une liaison simple, N(R¹), O, S, C(R¹)₂, Si(R¹)₂ ou B(R¹) ;
R¹ est pour chaque occurrence, de manière identique ou différente, H, F, CI, Br, I, CN, NO₂, Si(R²)₃, B(OR²)₂, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut être substitué par un radical ou par plusieurs radicaux R², où un ou plusieurs groupe(s) CH₂ non adjacents peut/peuvent être remplacé(s) par -C≡C-, Si(R²)₂, Ge(R²)₂, Sn(R²)₂, C=O, C=S, C=Se, C=NR², -O-, -S-, -COO- ou -CONR²- et où un ou plusieurs atome(s) de H peut/peuvent être remplacé(s) par F, CI, Br, I, CN ou NO₂, ou un système de cycle aromatique ou hétéroaromatique qui comporte de 5 à 40 atomes de cycle aromatique, lequel peut être substitué par un radical ou par plusieurs radicaux R¹ non aromatique(s), ou un groupe aryloxy ou hétéroaryloxy qui comporte de 5 à 40 atomes de cycle aromatique, lequel peut être substitué par un radical ou par plusieurs radicaux R¹ non aromatique(s), ou une combinaison de ces systèmes ; deux substituants R¹ ou plus peuvent ici également former un système de cycle monocyclique ou polycyclique l'un avec l'autre ou les uns avec les autres ;
R² est pour chaque occurrence, de manière identique ou différente, H ou un radical hydrocarbone aliphatique ou aromatique qui comporte de 1 à 20 atome(s) de C ;
X¹, X⁴ sont pour chaque occurrence, ce manière identique ou différente, un pont qui, avec Ar¹ et Ar², forme un système de cycle qui est sélectionné parmi C(R¹)₂, Si(R¹)₂, C=O, C=NR¹, C=C(R¹)₂, O, S, S=O, SO₂, N(R¹), P(R¹), P(=O)R¹, P(=S)R¹, B(R¹) ou une combinaison de deux, trois ou quatre de ces groupes ;
X², X³ sont pour chaque occurrence, de manière identique ou différente, un pont qui, avec Ar² et Ar³, forme un système de cycle cyclique qui est sélectionné parmi B(R¹), C(R¹)₂, Si(R¹)₂, C=O, C=NR¹, C=C(R¹)₂, O, S, S=O, SO₂, N(R¹), P(R¹), P(=O)R¹, P(=S)R¹ ou une combinaison de deux, trois ou quatre de ces groupes ;
p, n, o p est zéro et l'un des deux indices n ou o est égal à l'unité, tandis que l'autre des deux indices n ou o est zéro, n = 0 ou o = 0 ou p = 0 ici signifie que deux H ou radicaux R¹ sont présents en lieu et place du pont ;
q, r sont pour chaque occurrence 1 si l'atome central correspondant du groupe Y ou Z est un élément qui provient du 5ième groupe principal, et sont pour chaque occurrence égaux à 0 si l'atome central correspondant du groupe Y ou Z est un élément qui provient du 4ième ou du 6ième groupe principal ;
s est 1, 2 ou 3 ;
t est pour chaque occurrence, de manière identique ou différente, 0 ou 1, où t = 0 signifie que des radicaux R¹ sont liés en lieu et place du groupe E ; en outre, t = 0 si q=0.

2. Composés selon la revendication 1, **caractérisés en ce que** les symboles Y, Z représentent, de manière identique ou différente, azote, C=O, phosphore ou P=O.

3. Composés de la formule (1a) selon la revendication 2 : 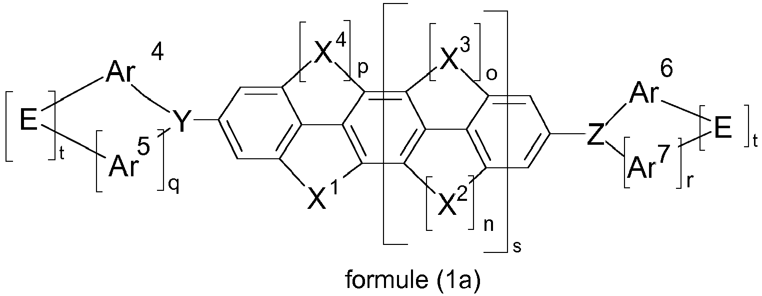 dans laquelle les symboles et indices présentent les mêmes significations que celles qui ont été décrites selon la revendication 1.

4. Composés selon une ou plusieurs des revendications 1 à 3, **caractérisés en ce que** les symboles Ar⁴, Ar⁵, Ar⁶, Ar⁷ représentent, de manière identique ou différente pour chaque occurrence, un système de cycle aromatique ou hétéroaromatique qui comporte de 5 à 16 atomes de cycle aromatique, triarylamine ou spirobifluorène, lequel peut dans chaque cas être substitué par un radical ou par plusieurs radicaux R¹.

5. Composés selon une ou plusieurs des revendications 1 à 4, **caractérisés en ce que** le symbole R¹ représente, de manière identique ou différente pour chaque occurrence, H, F, CN, un groupe alkyle en chaîne droite qui comporte de 1 à 5 atome(s) de C ou un groupe alkyle ramifié qui comporte de 3 à 5 atomes de C, où, dans chaque cas, un ou plusieurs groupe(s) CH₂ non adjacents peut/peuvent être remplacé(s) par -C≡C-, -O- ou -S- et où un ou plusieurs atome(s) de H peut/peuvent être remplacé(s) par F, ou un groupe aryle ou hétéroaryle monovalent qui comporte de 2 à 16 atomes de C, lequel peut être substitué par un radical ou par plusieurs radicaux R² ; deux radicaux R¹ ou plus ici peuvent également former un système de cycle l'un avec l'autre ou les uns avec les autres.

6. Composés selon une ou plusieurs des revendications 1 à 5, présentant les structures des formules (1b) et (1c) : 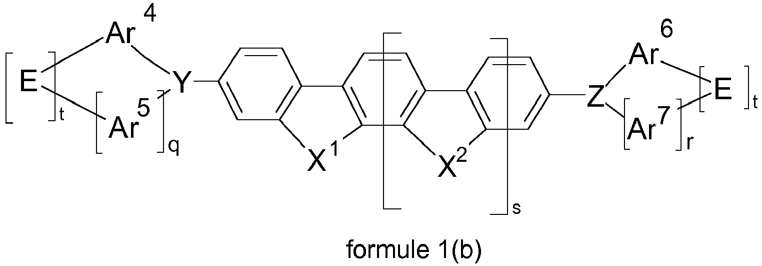 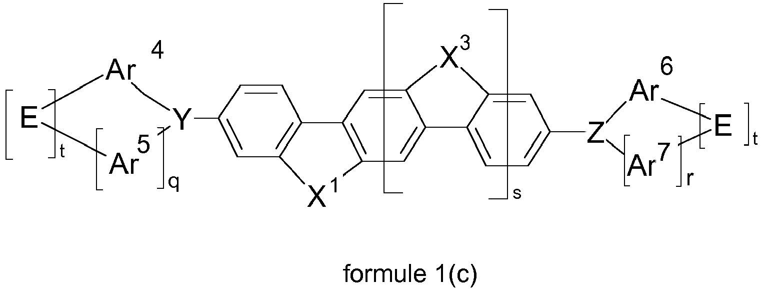 dans lesquelles les symboles et indices présentent les mêmes significations que celles qui ont été décrites selon la revendication 1.

7. Composés selon une ou plusieurs des revendications 1 à 6, **caractérisés en ce que** les symboles X¹, X², X³ et X⁴, pour chaque occurrence, de manière identique ou différente, représentent un pont qui, avec Ar¹ et Ar² ou avec Ar² et Ar³, forme un système cyclique qui est sélectionné parmi C(R¹)₂, C=O, C=NR¹, O, S, S=O, SO₂, N(R¹), P(R¹), P(=O)R¹, C(R¹)₂-C(R¹)₂,
C(R¹)₂-C(R¹)₂-C(R¹)₂, C(R¹)₂-O et C(R¹)₂-O-C(R¹)₂.

8. Composés de la formule (1d) selon la revendication 7 : 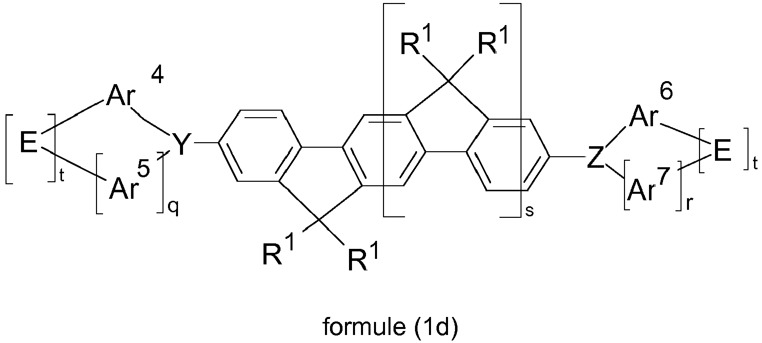 dans laquelle les symboles et indices présentent les mêmes significations que celles qui ont été décrites selon la revendication 1.

9. Composés selon la revendication 8, **caractérisés en ce que** les radicaux R¹ forment un système de cycle les uns avec les autres.

10. Composés selon une ou plusieurs des revendications 1 à 9, **caractérisé en ce que** Y = Z.

11. Composés selon une ou plusieurs des revendications 1 à 10, sélectionnés parmi les structures (1) à (104).

12. Utilisation de composés selon une ou plusieurs des revendications 1 à 11 dans des dispositifs électroniques organiques.

13. Dispositif électronique organique comprenant au moins un composé selon une ou plusieurs des revendications 1 à 11.

14. Dispositif électronique organique selon la revendication 13, sélectionné parmi le groupe qui est constitué par les dispositifs électroluminescents organiques (OLED), les transistors à effet de champ organiques (O-FET), les transistors à film mince organiques (O-TFT), les transistors à émission de lumière/électroluminescents organiques (O-LET), les circuits intégrés organiques (O-IC), les cellules solaires organiques (O-SC), les dispositifs à extinction de champ organiques (O-FQD), les cellules électrochimiques à émission de lumière/ électroluminescentes (LEC), les photorécepteurs organiques ou les diodes laser organiques (O-laser).

15. Dispositif électroluminescent organique comprenant une anode, une cathode et au moins une couche d'émission, **caractérisé en ce que** la couche d'émission comprend au moins un composé selon une ou plusieurs des revendications 1 à 11.

16. Dispositif électroluminescent organique selon la revendication 15, **caractérisé en ce qu'**un composé selon une ou plusieurs des revendications 1 à 11 est utilisé en tant qu'émetteur, et le matériau hôte de la couche d'émission est sélectionné au sein des classes des oligoarylènes, des oligoarylènes qui contiennent des groupes aromatiques condensés/fusionnés, des oligoarylènevinylènes, des complexes de métaux polypodaux, des composés de conduction de trous, des composés de conduction d'électrons, des cétones, des oxydes de phosphine, des sulfoxydes ou des atropisomères.

17. Dispositif électroluminescent organique selon la revendication15 ou 16, **caractérisé en ce que** d'autres couches sont présentes, lesquelles sont sélectionnées parmi une couche d'injection de trous, une couche de transport de trous, une couche de blocage de trous, une couche de transport d'électrons et/ou une couche d'injection d'électrons.

18. Dispositif électroluminescent organique selon une ou plusieurs des revendications 13 à 17, **caractérisé en ce que** le composé selon une ou plusieurs des revendications 1 à 11 est utilisé en tant que matériau de transport de trous dans une couche de transport de trous et/ou dans une couche d'injection de trous, et **en ce que** le composé dans ces couches peut en option être dopé au moyen de composés accepteurs d'électrons.

19. Dispositif électroluminescent organique selon une ou plusieurs des revendications 13 à 18, **caractérisé en ce que** le composé selon une ou plusieurs des revendications 1 à 11 est utilisé en tant que matériau de transport d'électrons dans une couche de transport d'électrons et/ou en tant que matériau de blocage de trous dans une couche de blocage de trous et/ou en tant que matériau de matrice triplet dans une couche d'émission.
